# EUROPEAN PATENT APPLICATION

(11) **EP 4 459 284 A1**
(43) Date of publication of application: **06.11.2024**
(21) Application number: 24174226.1
(22) Date of filing: 03.05.2024
(51) Int. Cl.: G01N 33/543

(54) **SENSOR ASSEMBLY**

(30) Priority: 04.05.2023 US 202318312075
(71) Applicant: Analog Devices International Unlimited Company, Limerick, V94 N4V2 (IE)
(72) Inventor: Berney, Helen, Limerick, V94 RT99 (IE); Lloyd, David, Wilmington, Massachusetts, 01887 (US)
(74) Representative: Elkington and Fife LLP

(57) **Abstract**

The present disclosure provides systems and method for determining a property of an analyte in a sample, the systems and methods use a sensor assembly comprising a sensing element. The method and systems remove at least a portion of non-specifically-bound species from the sensing element by applying an electric field to the sample matrix, wherein the electric field is configured to remove the non-specifically-bound species but has a strength less than that required to detach any specifically-bound analyte from the sensing element.

## Description

### FIELD OF THE DISCLOSURE

This disclosure relates to a method for determining a property of an analyte in a sample matrix and a system, for example a biosensor or chemical assay system, for sensing an analyte in a sample.

### BACKGROUND

Various biological and chemical assays are known for sensing analytes. Analytes may, for example, include biomarkers, such as hormones, established to assist in patient monitoring and/or diagnosis.

Many of these assays are now carried out using electrical sensors, such as electrochemical sensors. For example, some traditional sensors in these fields use sensing elements, such as those with planar sensing surfaces, which have immobilized anchor species provided thereon. These are typically provided on the surfaces of the sensing elements through covalent chemical linkage, covalent-like interactions (e.g. chemisorption of anchor species onto the surface through chemical bond formation) and non-covalent-like interactions (e.g. physisorption of anchor species onto the surface through weaker, often van der Waals, interactions) depending on the identity of the surface and the anchor species. These species are used to capture target analytes, with the binding of analytes causing a change in a measured output (e.g. current).

Although rapid improvements in the selectivity and sensitivity of these sensors have been made over the past decades, there is still a need to improve these, particularly for biological sensing systems. For example, there is a desire to increase the accuracy of the detection of a particular analyte, particularly where other non-analyte species may interfere with the measurement. This can occur, for example, through non-specific binding of non-analyte species (e.g. absorption on the surface of the sensing element) which can interfere with the measurement signal. There can be interference in the form of non-analyte species inhibiting binding of the analyte to the capture species. These issues impact limits of detection, signal-to-noise and speed of measurement, for example. Such problems can be particularly problematic in biosensors, for example where biological fluids are used as the same (e.g. blood, saliva, etc) as these contains numerous species, including large species such as proteins and ion complexes.

More generally, there is a desire to obtain a measurement more quickly as time to binding can be slow. Often an incubation period is required, slowing down measurements and limiting applications (such as point-of-care testing).

### SUMMARY OF THE DISCLOSURE

The present disclosure provides systems and method for determining a property of an analyte in a sample, the systems and methods use a sensor assembly comprising a sensing element. The method and systems remove at least a portion of non-specifically-bound species from the sensing element by applying an electric field to the sample matrix, wherein the electric field is configured to remove the non-specifically-bound species but has a strength less than that required to detach any specifically-bound analyte from the sensing element.

In a first aspect, there is provided a method for determining a property of an analyte in a sample, the sample comprising the analyte, non-analyte species and a sample matrix in which the analyte and non-analyte species are contained, the method comprising:
providing a sensor assembly, the sensor assembly comprising a sensing element comprising a capture species configured to specifically bind with the analyte, the sensing element providing a measurement signal indicative of the interaction of the sensing element with the sample;
providing the sample to the sensing element, wherein at least a portion of analyte and/or non-analyte species in the sample non-specifically bind to the sensing element and wherein least a portion of analyte specifically-binds to the sensing element;
obtaining a sample baseline measurement based on the measurement signal;
removing at least a portion of the non-specifically-bound analyte and/or non-analyte species from the sensing element by applying an electric field to the sample matrix, the electric field configured to remove non-specifically-bound analyte and/or non-analyte species from at least a portion of the sensing element but having a strength less than that required to detach specifically-bound analyte from the sensing element; and
determining the property of the analyte in the sample based on the measurement signal after the step of removing at least a portion of the non-specifically-bound analyte and/or non-analyte species from the sensing element.

In a second aspect, there is a system for determining a property of an analyte in a sample, the sample comprising the analyte, non-analyte species and a sample matrix in which the analyte and non-analyte species are contained, the system comprising:
a sensor assembly comprising a sensing element, the sensing element comprising a capture species configured to specifically bind with the analyte and the sensing element providing a measurement signal indicative of the interaction of the sensing element with the sample;
a sample manipulation device for manipulating analyte and/or non-analyte species on the sensing element, the sample manipulation device comprising a plurality of electrodes configured to apply an electric field about the sensing element;
a control unit configured to operate the sample manipulation device; and
a property determination unit configured to determine the property of the analyte;
wherein the control unit is configured to operate the sample manipulation device to generate the electric field so as remove any non-specifically-bound analyte and/or non-analyte species but retain specifically-bound analyte on the sensing element;
wherein the property determination unit is configured to determine a sample baseline measurement based on the measurement signal before and/or during operation of the sample manipulation device; and
wherein the property determination unit is configured to determine the property of the analyte based on the measurement signal after operation of the sample manipulation device.

### BRIEF DESCRIPTION OF THE DRAWINGS

The present invention will now be described in more detail with reference to the accompanying drawings, which are not intended to be limiting:
Figs. 1A to 1E provide a schematic cross-sectional view of a system according to an embodiment;
Fig. 2 provides a block diagram of a method according to an embodiment;
Fig. 3 provides a block diagram of a method according to an embodiment;
Figs. 4A to 4D provide example applied voltage schemes for a removal step according to an embodiment;
Fig. 5 provides an example applied voltage scheme according to an embodiment;
Figs. 6A to 6D provide schematic cross-sectional views of a part of a system according to an embodiment;
Figs. 7A and 7D provide an example applied voltage and a corresponding signal response for embodiments, respectively;
Figs. 8A to 8D provide schematic cross-sectional views of a part of a system according to an embodiment;
Fig. 8E provides an example signal response;
Fig. 9 provides a schematic cross-sectional plan view of a system according to an embodiment;
Fig. 10 provides a schematic plan view of a system according to an embodiment; and
Fig. 11 provides a schematic cross-sectional view of the system of Fig. 10.

### DETAILED DESCRIPTION

Electrochemical sensors, including biosensors and chemisensors, are used for various biological and chemical assay are known for sensing analytes in samples. Analytes may, for example, include biomarkers, such as hormones, established to assist in patient monitoring and/or diagnosis.

Although rapid improvements in the selectivity and sensitivity of these sensors have been made over the past decades, there is still a need to improve the selectivity and sensitivity, particularly for biological sensing systems. For example, most samples, particularly biological samples, will comprise a sample matrix and a number of components within the sample matrix, including potentially the analyte and/or some non-analyte species. Non-specific binding of non-analyte species in the sample can occur (e.g. absorption on the surface of the sensing element) which can also cause a change in the measured output or can impede binding of analyte to the capture species. Similarly, non-specific binding of the analyte to the surface (rather than the capture species) can also provide a response which is difficult to distinguish from non-analyte species in typical measurements. This is particularly the case for samples such as biological fluids, for example, which contain numerous contaminants or components which can compete for binding sites (e.g. proteins, ions) and can slow measurements down. Cross-reactivity can also be a problem which interferes with the signal.

Some strategies to address non-specific binding can include using a blocking buffer, crowding the sensing surface with other species or modifying the surface. However, these require additional components in the system and can be limited in their versatility, requiring designing and testing for each specific to assays. Others use modified capture species, but this but this can be a costly and inefficient process and will add complexity to the manufacture processes while also reducing the versatility of the ultimate system.

In one embodiment, a method for determining a property of an analyte in a sample, the sample comprising the analyte, non-analyte species and a sample matrix in which the analyte and non-analyte species are contained, the method comprising:
providing a sensor assembly, the sensor assembly comprising a sensing element comprising a capture species configured to specifically bind with the analyte, the sensing element providing a measurement signal indicative of the interaction of the sensing element with the sample;
providing the sample to the sensing element, wherein at least a portion of analyte and/or non-analyte species in the sample non-specifically bind to the sensing element and wherein least a portion of analyte specifically-binds to the sensing element;
obtaining a sample baseline measurement based on the measurement signal;
removing at least a portion of the non-specifically-bound analyte and/or non-analyte species from the sensing element by applying an electric field to the sample matrix, the electric field configured to remove non-specifically-bound analyte and/or non-analyte species from at least a portion of the sensing element but having a strength less than that required to detach specifically-bound analyte from the sensing element; and
determining the property of the analyte in the sample based on the measurement signal after the step of removing at least a portion of the non-specifically-bound analyte and/or non-analyte species from the sensing element.

Removing or displacing at least a portion of the non-specifically-bound analyte and/or non-analyte species from the sensing element ("removal step") can provide significant sensitivity improvements by reducing the signal-to-noise ratio (SNR). Specifically, the removal step strips away at least a portion of the non-specifically-bound species from the surface(s) of the sensing element thereby reducing or eliminating the interference from these on the measurement signal. Therefore, compared to methods in which samples are left to incubate and subsequently the response is measured without any compensation or means to deal with non-specific binding, the ultimate measurement will be more accurate and limits of detection are reduced.

Moreover, the removal step can also have the benefit of improving the response time of the sensor. Non-specific binding on a sensing element can lead to interference with specific binding processes and require longer incubation times. By disrupting species that are non-specifically-bound, this interference is removed. As an example, this is important in systems where binding kinetics are monitored because binding is influenced and slowed by species adsorbed on the surface.

By using an electric field to perform removal, it also avoids drawbacks associated with other methods which seek to avoid non-specific binding, such as having to test different blocking buffers, blocking layers or capture species. Instead, the same fundamental sensor structure, which can be relatively straightforward in structure, can be used in numerous different arrangements with minimal or no modification. This system also provides flexibility within the same system to deal with unexpected responses. It will be appreciated that it will not always be possible to know what species with non-specifically bind with the sensing element, and whether alternative means that seek to prevent any non-specific binding can work. An electric field can be reconfigured or modified in a straightforward manner to tailor the removal step and the success can be monitored simply by measuring the usual response provided by the sensor assembly. This provides versatility that different chemical-based solutions to non-specific binding could not.

### Sample baseline measurement, adjustment and compensation

Obtaining a sample baseline measurement based on the measurement signal can be carried out at least before and/or during the removal step (e.g. during the initial stages of the removal step). That is, the method may comprise obtaining a sample baseline measurement based on the measurement signal and, subsequently and/or sequentially, performing the removal step. This provides an indication of the signal before the removal of the non-specifically-bound components. For example, this may obtained before or simultaneously with the start of the removal step (e.g. the application of the electric field), or shortly thereafter (e.g. within the first 20% of the duration of the removal step).

By obtaining a sample baseline measurement prior to or at the start of the removal step, this information can then be used to correct further data or compensate for the non-specifically-bound species. The sample baseline (or background) measurement, at least at the beginning, corresponds to the influence of the non-specific binding on the total measurement signal. This enables the determination of what part of the signal is attributable to the non-specific binding, which in turn can be used to improve the speed and sensitivity of future measurements where the removal step need not occur. For example, further measurements using the sensor assembly (discrete, or later in a continuous process) may forgo the removal step but still benefit from an improved SNR or sensitivity due to the data. Moreover, it can be used to determine further information about the sample. In other examples, the determination of the property of the analyte may be based in part on the sample baseline measurement (as a corrective factor, for example). Alternatively or additionally, the sample baseline measurement may be used to determine at least one parameter of the step of removing at least a portion of the non-specifically-bound species from the sensing element by applying an electric field to the sample matrix. For example, at least one of the duration of the removal step or the parameters of the electric field, such as at least one of the duration of the application of the electric field to the sample matrix, the strength of the electric field, and/or the number of repetitions of the application of the electric field.

This sample baseline measurement may also be determined based on the measurement signal during the removal step. Information about the kinetics and nature of the non-specific binding can be determined during this process.

In one embodiment, the sample baseline measurement is used to determine at least one parameter of the step of removing at least a portion of the non-specifically-bound analyte and/or non-analyte species from the sensing element. In this way, the sample baseline measurement may provide information on the nature of non-specific binding and this can be used to optimise the removal step.

In one embodiment, the method further comprises determining an adjustment factor based on the measurement signal during the step of removing at least a portion of the non-specifically-bound analyte and/or non-analyte species from the sensing element and after the sample baseline measurement, and further comprises adjusting at least one parameter of the step of removing at least a portion of the non-specifically-bound analyte and/or non-analyte species from the sensing element based on the adjustment factor. This may be based on the measurement signal during or after the application of the electric field. The adjustment factor provides a means for actively adjusting the removal step mid-process (e.g. by allowing for the shortening of the electric field application, providing for further applications or by allowing for the provision of further removal steps). It provides feedback on the current situation with respect to the non-specific binding (essentially an intermediate measurement after a period time has passed since the application of the electric field and sample baseline measurement) relative to the sample baseline measurement. The adjustment factor may also determine that the removal step can be halted. For example, the removal step may be continued until the adjustment factor or intermediate sample measurement, if taken, indicates that a threshold is reached, indicating that sufficient non-specific binding species have been removed.

In one further embodiment, the adjustment factor is based on the rate of change in the measurement signal during the step of removing at least a portion of the non-specifically-bound analyte and/or non-analyte components from the sensing element. That is, the sample baseline measurement may be obtain for at least a part of the duration of the removal step, and may also take into account the measurement signal before the removal step. This can be useful as the amount of signal attributable to non-specific binding may not be known, but the rate of change may provide an indication of the effectiveness of the process. For example, once the rate of change has slowed, it may be that the removal step can be halted. Conversely, in other cases, it may indicate that the intensity or strength of the electrical field should be increased (e.g. to remove species that have a stronger non-specific affinity). The approach may depend on the sample type. Additionally or alternatively, the rate of change may provide information on the non-specifically-bound species, for example by looking at the kinetics and interaction. For example, electrophoresis is known to be affected by the size of the components and the charge, such that the rate of change of signal as the removing step occurs. This can provide useful information about the nature of the other non-analyte components, and can enable more accurate filtering of the signals associated with such non-analyte components.

In one embodiment, the method further comprises, subsequent to determining a property of the first sample mentioned above, providing a second sample to the sensor assembly; and determining the property of the analyte in the second sample based on the measurement signal for the second sample and a signal compensation factor, wherein the signal compensation factor is based on the sample baseline measurement and the measurement signal for the first sample. In this way, the sensor assembly can be used to take an initial measurement corresponding to interference from non-specific binding, and subsequent measurements can be corrected using the compensation factor without having to repeat the removal step. This speeds up subsequent measurements. The signal compensation factor is based on the sample baseline measurement from the first measurement cycle and the measurement signal for the first sample. This signal compensation factor may also be based in part on the further data measured in the methods disclosed herein, such as that obtained for the adjustment factor and resulting from the other manipulation processes.

It will be appreciated that, in embodiments, measurement may be of discrete or continuous samples. Thus, providing a second sample may comprise providing a separate sample or may relate to a sample at a later time period in a continuous process.

In one embodiment, the sample baseline measurement is obtained at least during the step of removing at least a portion of the non-specifically-bound analyte and/or non-analyte species from the sensing element, and the sample baseline measurement comprises the rate of change in the measurement signal. The rate of change can provide useful information about the kinetics and nature of the non-specific binding during the process. For example, the information over time and changes in the rate may give information about the types of species bound to the sensor element. This may give rise to a specific fingerprint or pattern, providing further information on the species and in turn how to compensate or obtain an accurate reading.

### Electric Field

The electric field used during the removal step is applied and configured so as to remove non-specifically-bound species from at least a portion of the sensing element but has a strength less than that required to detach specifically-bound analytes from the sensing element. That is, it is arranged relative to the sensing element (e.g. adjacent and so that the electric field can influence sample on the sensing element (this may be the field overlaps with at least one surface of the sensing element or has sufficient force to create a movement on the surface of the sensing element)) and has a strength or intensity that is sufficient to remove non-specifically-bound components from at least a portion of (or in some embodiments all) of the sensing element but less than that required to detach specifically-bound analytes from the sensing element. It therefore produces a force which acts on the species within the sample that is sufficient to move at least some of the non-specifically-bound analytes away from the surface of the sensing element but less than that required to detach specifically-bound analytes from the sensing element (a force which is lower than the force corresponding to the affinity between the capture species and the analyte).

Although the electric field discussed above and the embodiments mentioned below are used in respect of the removal step, as will become apparent embodiments of the systems, sensor assemblies and methods disclosed herein may use an electric field in other operations which involve manipulation of the species within the sample matrix, including the analyte. The features disclosed in respect also apply to the electric field used in any part of the disclosed methods unless indicated otherwise.

In embodiments, the strength or intensity of the electric field is varied. This can be e.g. during the removal step or during other manipulation steps (including detachment of the analyte). Thus, in one embodiment, the strength of the electric field is varied during the step of removing at least a portion of the non-specifically-bound analyte and/or non-analyte components from the sensing element. In other words, the strength or intensity (e.g. as measured by the input voltage (V/m or mV/m)) of the electric field is varied. This will provide a different response than if a single value electric field is applied and, if measurements are recorded, can generate additional information on the types of non-specific binding or the state of the sensor assembly.

For example, the electric field may vary between a minimum and a peak or maximum strength, the peak strength being a strength which is sufficient to remove non-specifically-bound species from at least a portion of the sensing element but has a strength less than that required to detach specifically-bound analytes from the sensing element. This can be achieved by varying the voltage across the electrodes used to generate the field, for example, or could be achieved by other means such as moving the relative position of the electrodes.

In some embodiments, this variation of the electric field may be selected so as to cause movement of the species within the sample matrix in a single direction. For example, changing from a first value imparting a first directional movement to a second value imparting the same directional movement could be used (e.g. the application of a first positive voltage across the electrodes generating the field followed by a second positive voltage, or the inverse (negative to negative)).

In other embodiments, the variation may be selected so as to cause movement of the species within the sample matrix in plural directions, for example opposite directions. For example, the application of a first positive voltage across the electrodes generating the field followed by a second negative voltage, or the reverse. These can be used to further interrogate the measurement signal and may improve removal depending on the nature of the binding on the surface. Where opposing directions are cycled, numerous methods within these can be utilized to ensure removal away from the sensing element if required, such as additional applications in one direction, or asymmetric intensities or durations of the application of the electric field.

In alternative or additional embodiments, the electric field may be varied between positive and negative input voltages so as to vary the properties of the electric field.

In embodiments, the electric field may be varied by at least one of the following methods: a linear ramp or sweep where the intensity is increased at a steady rate (e.g. 1 mV/m/s), a non-linear ramp or sweep where the intensity is increased at varying rate(s), at least one step change increase, or combinations thereof. Each can be between the whole range or a part of the range between the minimum and maximum. Where it is the former, these can be combined in a single step of varying. Alternatively, each could be employed in multiple separate applications of an electric field. Each may lead to a different response thereby providing different information on the non-specific binding. For example, different approaches may lead to different detachments and/or interactions between the sensing element and the species.

The force applied and the rate of the rate of change of the force applied may be directly linked to the probability that the species in question (e.g. the non-specifically bound species or, where specifically-bound analyte is subsequently removed, the specifically-bound species) can be detached from the analyte.

In some embodiments where the force applied is varied, the method may comprise applying a first force (which may be zero) and increasing or decreasing the applied force to a second, different force. Where this is the removal step, this comprises increasing the applied force from a first force to a second force. This may be a continuous change from the first force to the second force or may comprise a step change through the range from the first force to the second force. In embodiments, the first force may be from 0 to 20 pN (such as 0 to 10 pN or 0 to 5 pN) and the second force is greater than the first force. In embodiments, the second force may be at least 10 pN, such as least 20 pN, at least 30 pN, at least 40 pN or at least 100 pN. For example, from 10 to 400 pN, such as 30 to 400 pN, such as 30 to 300 pN. Accordingly, embodiments may comprise increasing the applied force from a first force of from 0 to 20 pN to a second force, which is a greater than the first force and is at least 10 pN, such as 20 to 400 pN. Varying the force applied through a ramp or a sweep within these force ranges can successfully and selectively detach non-specifically bound components.

The rate of change of the applied force can influence when detachment of species occurs (e.g. during a removal step or during a detachment step). A slower rate of change may lead to more bonds breaking at a lower absolute force. Higher force rates of change may lead to more bonds breaking at higher absolute breaking force. In some embodiments, increasing or decreasing the applied force from the first force to the second force may comprise increasing or decreasing the force at a rate of from 0.01 pN/s to 200 pN/s, such as from 0.01 pN/s to 100 pN/s, from 0.01 pN/s to 50 pN/s, from 0.02 pN/s to 200 pN/s, from 0.02 pN/s to 50 pN/s or from 0.02 pN/s to 10 pN/s. This can be the average rate of change, such as a mean rate of change across the range of the first force to the second force, for example where the changes are non-linear or stepped. In some embodiments, the force is selected so as to change the k_{off} value for the interaction in question (e.g. the removal of non-specifically bound species) by a factor of at least 2, such as at least 10.

In embodiments, the removal step and/or manipulation steps may comprise cycling the respective force (e.g. electric field) between at least two strengths or intensities. In one embodiment, this can be between on and off. In other embodiments, this may be between cycled between at least two values. In some embodiments, these may be values imparting only a single direction of movement on the species (e.g. a field in the same direction, for example between two fixed electrodes and of the same polarity). In other embodiments, the values may impart different directions, such as opposing directions of movement on the species (e.g. different fields in different directions, for example between two fixed electrodes in which the polarity is switched). In embodiments where the non-specifically-bound species removed in the removal step are moved towards a surface of the sensing element, this may be followed by an additional removal step (according to any of the removal steps set out herein).

Such methods can be used to obtain additional information about the non-specifically-bound species, which can be further used to improve the knowledge about the non-specific binding species. For example, rate of change in the measurement signal could be monitored. This can in turn be used in compensating for the non-specific binding.

It will be appreciated that in any of the embodiments mentioned here, although the resultant force of the electric field acting on a particular component (e.g. the analyte or non-analyte species) will depend on a number of factors, including the charge on the component, the magnitude of the force will be determined by the magnitude of the electric field such that a higher V/m value will lead to a greater force acting on the species in the sample.

Generation of the electric field can be achieved by applying a voltage across a first electrode and a second electrode located adjacent to (e.g. next to or abutting) the sensing element. The sample (e.g. fluid, such as a solution) can be received between the electrodes. In one embodiment, during the step of removing at least a portion of the non-specifically-bound components from the sensing element, the electric field is generated between by applying a voltage across at least a first electrode and a second electrode located adjacent the sensing element.

In embodiments, during the removal step, the voltage across the first electrode and second electrode is from -2V to 2V, such as -1.5V to 1.5V. This may exclude 0V. For example, in certain embodiments, the voltage (i.e. potential difference) can be greater than 0 to 2V, such as greater than 0 to 1.5V, greater than 0 to 1V, greater than 0 to 750mV, greater than 0 to 500 mV or0 to 350 mV. This can be the magnitude (i.e. the absolute value exclusive of polarity), such that, where negative, "greater than 0 to 1.5V" is equivalent to "less than 0 to -1.5V". These can be a DC voltage. As set out above, this can be at least one discrete value within these ranges, or may comprise a ramp or step through these ranges.

As set out above, the applied force can be provided by changing the voltage applied to the system. In embodiments, the voltage across the first electrode and second electrode can be varied to provide the change in force. Accordingly, in embodiments, the voltage applied may be increased from a first voltage to a second voltage. This may be a continuous change from the first voltage to the second voltage or may comprise a step change through the range. In embodiments, the first voltage is less than the second voltage and the method and system may increase the voltage from the first voltage to the second voltage. This can be the magnitude of the voltage - i.e. the absolute number irrespective of polarity. In some embodiments, the first voltage can be from 0V to 150mV, such as 0V, and the second voltage can be at least 250mV or at least 500mV or at least 750mV, at least 1V. In embodiments, the second voltage may be from 250mV to 2V, such as from 250mV to 2V, 250 mV to 1.5V, 500mV to 1.5V or from 250mV to 1V. The difference between the first voltage and the second voltage may be at least 250mV or at least 500mV or at least 750mv.

In some embodiments, increasing the voltage from the first voltage to the second voltage may comprise increasing the voltage at a rate of from 0.1mV/s to 20V/s, such as from 0.25mV/s to 10V/s, from 0.1mV/s to 1V/s, from 0.1mV/s to 250mV/s, or from 0.1mV/s to 10mV/s. This can be the average rate of change, such as a mean rate of change across the range from the first voltage to the second voltage, for example where the changes are non-linear or stepped.

In embodiments, the removal step may be repeated for the same sample under different applied force conditions, for example using different rates of change of applied force or applied voltage. This can be different rates of change when increasing or decreasing the applied force from the first force to the second force. Modifying the force applied can lead to a different responses for each of the types of binding and types of species, which in turn can provide additional information about the kinetics and nature of the non-specific and specific binding. For example, in some embodiments, the method may comprise a first removal step at a rate of change from a first applied force to a second applied force and the method may further comprises a second removal step at a first rate of change from a third applied force to a fourth applied force at a second rate of change. The first and third applied forces may be the same and the second and fourth applied forces may be the same. The first and second rate of changes may be different. In embodiments where a sample baseline is measured, the sample baseline may be determined based on the measurement signal during the first and second removal steps. In some embodiments, the selection of the second rate of change may be dependent on the response from the measurement signal from the first rate of change. As such, a first response can be used to determine an appropriate second rate of change.

Detaching the analyte from the capture species (e.g. in a detachment step) or a tag or detection element from the analyte requires a force to be applied which is sufficient to overcome the strength of the interaction between these elements. This force applied will depend on the net electric charge of the species it is acting on, and the force required to detach the relevant species will depend on the interaction between the species, such as strength of the bond. These forces are typically in the range of 10 to 400 pN, such as 30 to 400 pN, such as 30 to 300 pN. Accordingly, in embodiments, during a detachment step, the force applied to the bound analyte is from of 10 to 400 pN. The force applied during the other steps, such as manipulation or removal steps (which may be applied by means other than electric fields), can accordingly be less than this, such as less than or equal to 50 pN, less than or equal to 40 pN, less than or equal to 30 pN or less than or equal to 10 pN. It will be appreciated that a higher force can be applied during manipulation or removal steps where the binding between the analyte and other species is higher. For example, where a 140 pN force is required to detach an analyte from a capture species, the force applied during manipulation or removal steps may be less than or equal to 100 pN. These forces can be provided by generating an electric field using the voltages mentioned above, for example, particularly in or immediately adjacent the through holes which will provide an electric field concentrating structure (e.g. at the edges of the through holes). As noted above, the force applied will depend on the net electric charge of the species it is acting on. This force applied will depend on the net electric charge of the species it is acting on. For example, in some embodiments, the electric field may require a strength of from 0.5 × 10⁶ V/m to 1 × 10⁸ V/m, such as 1 × 10⁶ V/m to 2 × 10⁷ V/m. This may be present in the localised regions around the through holes and need not be throughout the entire applied field.

In some embodiments, other parameters of the electric field are varied, such as frequency.

### Specifically-bound Analyte Manipulation

The method may further include manipulation of the specifically-bound analyte on the sensing element or layer, including one of manipulation to cause movement or even manipulation to cause detachment. This can lead to additional information about the analyte, for example based on the response as the analyte moves on the sensing element. In some embodiments discussed below, the sensing element includes structures which influence the measurement signal and these manipulation techniques can be used to influence proximity of the specifically-bound analyte to these structures to gain further data, for example by moving them into or around these structures.

In one embodiment, the method further comprises manipulating at least a portion of the specifically-bound analyte after the step of removing at least a portion of the non-specifically-bound analyte and/or non-analyte components from the sensing element, wherein manipulating specifically-bound analyte comprises applying a force to the sample matrix sufficient to move specifically-bound analyte on the sensing element (referred to herein as a "manipulation step"); and determining the property of the analyte in the sample is based on the measurement signal during and/or after the step of manipulating specifically-bound analyte.

In one embodiment, the method further comprises detaching at least a portion of the specifically-bound analyte after the step of removing at least a portion of the non-specifically-bound analyte and/or non-analyte components from the sensing element, wherein detaching specifically-bound analyte comprises applying a force to the sample matrix sufficient to detach specifically-bound analyte from the sensing element (referred to herein as a "detachment step"); and wherein determining the property of the analyte in the sample is based on the measurement signal during and/or after the step of detaching the specifically-bound analyte.

In some embodiments, manipulation may comprise both a manipulation step and a detachment step. These may be distinct steps or performed in sequence, for example as a continuous increase in the force applied. In one embodiment, the specifically-bound analyte is first displaced or moved and subsequently the force is increased to the point at which the specifically-bound analyte is detached.

In one embodiment, determining the property of the analyte is based on the rate of change in the measurement signal during the manipulation step. Additionally or alternatively, determining the property is based on the rate of change in the measurement signal during the detachment step.

The forces used in the steps disclose above can be provided by any suitable means of generating a force which acts on the species to move them within the sample matrix on the sensing element or layer. For example, in some embodiments, this can be through the use of an electric field, magnetic field, ultrasound energy, thermal gradients, osmotic gradients, density gradients, and/or a fluid flow. In some embodiments, the force is provided by at least one of an electric field or a magnetic field (this may include an electromagnetic field), and may include both. Accordingly, in some embodiments, the sample manipulation device comprises a plurality of electrodes configured to apply an electric field about the sensing element. In other embodiments, thermal gradients, osmotic gradients and/or density gradients can be used, for example by two different regions having different properties in two different regions around the sensing layer (e.g. above and below).

Where an electric field is used in a manipulation or detachment step, the voltage used to generate the field is from -2V to 2V, such as -1.5V to 1.5V. This may exclude 0V and may be at least 250mV, at least 500mV, at least 750mV, at least 1V. For example 500 mV to 10 V, such as 500mV to 5V, such as from 1V to 5V. For example, where AC is used, this can be from 500 mV to 25 V, such as from 1Vto 25V, such as from 1V to 5V. Where DC is used, this can be from 500 mV to 1.5 V. This can be the magnitude (i.e. the absolute value exclusive of polarity), such that, where negative, "0 to 1.5V" is equivalent to "0 to -1.5V".

Detaching the analyte from the capture species (e.g. in a detachment step) or a tag or detection element from the analyte requires a force to be applied which is sufficient to overcome the strength of the interaction between these elements. This force applied will depend on the net electric charge of the species it is acting on, and the force required to detach the relevant species will depend on the interaction between the species, such as strength of the bond. These forces are typically in the range of 10 to 400 pN, such as 30 to 400 pN, such as 30 to 300 pN. Accordingly, in embodiments, during a detachment step, the force applied to the bound analyte is from of 10 to 400 pN. The force applied during the other steps, such as manipulation or removal steps, can accordingly be less than this, such as less than or equal to 50 pN, less than or equal to 40 pN, less than or equal to 30 pN or less than or equal to 10 pN. It will be appreciated that a higher force can be applied during manipulation or removal steps where the binding between the analyte and other species is higher. For example, where a 140 pN force is required to detach an analyte from a capture species, the force applied during manipulation or removal steps may be less than or equal to 100 pN. These forces can be provided by generating an electric field using the voltages mentioned above, for example, particularly in or immediately adjacent the through holes which will provide an electric field concentrating structure (e.g. at the edges of the through holes). As noted above, the force applied will depend on the net electric charge of the species it is acting on. This force applied will depend on the net electric charge of the species it is acting on. For example, in some embodiments, the electric field may require a strength of from 0.5 × 10⁶ V/m to 1 × 10⁸ V/m, such as 1 × 10⁶ V/m to 2 × 10⁷ V/m. This may be present in the localised regions around the through holes and need not be throughout the entire applied field.

As set out above for the removal step, the applied force applied during the detachment step may be varied. As such, in the same manner as the removal step, the method during the detachment step may comprise applying a first force (which may be zero) and increasing or decreasing the applied force to a second, different force. This may comprise increasing the applied force from a first force to a second force. This may be a continuous change from the first force to the second force or may comprise a step change through the range from the first force to the second force. In embodiments, the first force during the detachment step may be from 0 to 50 pN, such as 10 to 50pN or 10 to 40 pN, and the second force is greater than the first force. In embodiments, the second force may be at least 10 pN, such as least 20 pN, at least 30 pN, at least 40 pN or at least 100 pN. For example, from 30 to 400 pN, such as 50 to 400 pN, such as 50 to 300 pN. Accordingly, embodiments may comprise increasing the applied force from a first force to a second force, which is greater than the first force and is at least 20 pN, such as 20 to 400 pN. Varying the force applied through a ramp or a sweep within these force ranges can successfully and selectively detach non-specifically bound components.

As with the removal step, the voltage across the first electrode and second electrode can be varied to provide the change in force during the detachment step. Accordingly, in embodiments, the voltage applied may be increased from a first voltage to a second voltage. This may be a continuous change from the first voltage to the second voltage or may comprise a step change through the range. In embodiments, the first voltage is less than the second voltage and the method and system may increase the voltage from the first voltage to the second voltage. This can be the magnitude of the voltage - i.e. the absolute number irrespective of polarity. In some embodiments, the first voltage may be at least 250mV, at least 500mV, at least 750mV, at least 1V and the second voltage can be greater than the first and at least 250mV or at least 500mV or at least 750mV, at least 1V. In embodiments, the second voltage may be from 250mV to 20V, such as from 250mV to 10V, or 500mV to 5V. The difference between the first voltage and the second voltage may be at least 250mV or at least 500mV or at least 750mv.

The rate of change of the applied force can influence when detachment of species occurs (e.g. during a removal step). A slower rate of change may lead to more bonds breaking at a lower absolute force. Higher force rates of change may lead to more bonds breaking at higher absolute breaking force. In some embodiments, increasing or decreasing the applied force from the first force to the second force during the detachment step may comprise increasing or decreasing the force at a rate of from 0.01 pN/s to 200 pN/s, such as from 0.01 pN/s to 100 pN/s, from 0.01 pN/s to 50 pN/s, from 0.02 pN/s to 200 pN/s, from 0.02 pN/s to 50 pN/s or from 0.02 pN/s to 10 pN/s. This can be the average rate of change, such as a mean rate of change across the range of the first force to the second force, for example where the changes are non-linear or stepped. In some embodiments, the force is selected so as to change the k_{off} value for the interaction in question (e.g. the removal of specifically bound species) by a factor of at least 2, such as at least 10.

In some embodiments of the detachment step, increasing the voltage from the first voltage to the second voltage may comprise increasing the voltage at a rate of from 0.1mV/s to 20V/s, such as from 0.25mV/s to 10V/s, from 0.1mV/s to 1V/s, from 2.5mV/s to 500 mV/s, from 0.1mV/s to 250mV/s, or from 0.1mV/s to 10mV/s. This can be the average rate of change, such as a mean rate of change across the range from the first voltage to the second voltage, for example where the changes are non-linear or stepped.

In embodiments, the detachment step may be repeated for the same sample under different applied force conditions, for example using different rates of change of applied force or applied voltage. This can be different rates of change when increasing or decreasing the applied force from the first force to the second force. Modifying the force applied can lead to a different responses for each of the types of binding and types of species, which in turn can provide additional information about the kinetics and nature of the non-specific and specific binding. For example, in some embodiments, the method may comprise a first detachment step at a rate of change from a first applied force to a second applied force and the method may further comprises a second detachment step at a first rate of change from a third applied force to a fourth applied force at a second rate of change. The first and third applied forces may be the same and the second and fourth applied forces may be the same. The first and second rate of changes may be different. In embodiments, the determination of the property may be determined based on the measurement signal obtained during the first and second detachment steps. In some embodiments, the selection of the second rate of change may be dependent on the response from the measurement signal from the first rate of change. As such, a first response can be used to determine an appropriate second rate of change.

In some embodiments, the strength or intensity of the fields may provide a force that is at least 1.5 times stronger than the force provided by the electric field used in the removal step, for example at least 2 times, at least 3 times. This may be by the field strength or intensity being at least 1.5 times stronger than the intensity or strength provided by the electric field used in the removal step, for example at least 2 times, at least 3 times.

The removal step may be carried out for at least 0.1ms, such as at least 5 ms, 10 ms, at least 50 ms or at least 100 ms. This can be 0.1 ms to 5 minutes, for example. The manipulation or detachment steps, where present, may be carried out for 0.1ms, such as at least 5 ms, 10 ms, at least 50 ms or at least 100 ms, at least 1s or more. This can be 0.1 ms to 5 minutes, for example. Where the steps noted above are alternated/cycled (and may further include periods between where none occurs), the total period may be 0.1 ms to 30 minutes, such as 0.1ms to 10 mins, 0.1 ms to 5 mins, 1 ms to 5 mins or any suitable range therebetween. An incubation step, where present, may be carried out for at least 5s, such as at least 60 s. This can be up to 30 mins, or up to 10 mins, for example.

### Other analyte manipulation

In one embodiment, the step of providing the sample matrix to the sensing element comprises applying an electric field to the sample matrix on the sensing element, the electric field being configured so as to cause movement of the analyte towards the capture species. Such a method can speed up measurement time by causing movement of the species in the sample to migrate to the sensing element at a movement speed which is greater than diffusion. This can reduce the time of or reduce the need for an incubation period. This may also be used as a further filter of the sample, since different species will move at different rates and therefore may be selectively driven to (or beyond) the sensing element, also speeding up measurement time and accuracy.

### Analyte, Capture Species and Detection Species

The methods, systems and sensor assemblies disclosed herein can be used to determine or measure a property of an analyte in a sample, such as an analyte characteristic. In certain embodiments, this can be selected from the concentration of the analyte in the sample, the diffusion constant of the analyte (e.g. rate of diffusion measured in m²/s) in the sample matrix, or a combination thereof. The terms "analyte concentration" or "concentration of the analyte" as used herein may, in certain embodiments, refer to the activity of the analyte. The activity of the analyte may provide a measure of the effective concentration of the analyte in a sample matrix.

The analyte may, for example, be selected from a molecular species, a metal ion, a virus, and a microorganism. Biomolecule analytes are particularly useful and may, for instance, be a hormone selected from an eicosanoid, a steroid, an amino acid, amine, peptide, protein, a nucleic acid, single or double stranded DNA, peptide nucleic acid.

Any suitable analyte capture species can be selected, according to the analyte which is intended to be sensed by the sensor assembly. For example, the capture species may comprise an antibody with specificity for a particular antigen. In such an example, the analyte may take the form of the antigen. More generally, the capture species may, in some embodiments, comprise at least one selected from a protein, a peptide, a carbohydrate, a nucleic acid, and an aptamer. The protein may, for example, be an enzyme, such as an enzyme having specificity for the analyte. In other non-limiting examples, the protein is an antibody. In the latter case, the analyte may be an antigen which is specifically bound by the antibody. The capture species may, for instance, comprise or be defined by an antigen. In this case, the analyte may be a species, such as an antibody, which is specifically bound by the antigenic capture species. The antigen may be or comprise, for example, a protein, a peptide, a carbohydrate, such as a polysaccharide or glycan. The sensor assembly may comprise a plurality of capture species (i.e. plural of the same capture species).

The captures species may be a rigid or flexible species. In some embodiments, the capture species binds to the analyte via a capture moiety and is configured so that this moiety is moveable relative to the sensing element. In this way, the analyte can move relative to the sensing element under the action of a sufficiently strong force (e.g. electric field). This may be as a result of the capture species being a flexible species. For example, the capture species may comprise a first portion bound to a surface of the sensing element and a second portion comprising the capture moiety, the two portions separated by a flexible linker. Flexibility in this regard is sufficient such that a bound analyte can be moved so as to interact with an adjacent structure, such as into an adjacent through hole. In some embodiments, the capture species comprises a double stranded DNA linker, the linker comprising a chain length of at least 24-mer, such as at least 25-mer, such as at least 30-mer.

In an embodiment, the analyte capture species comprises an aptamer. An aptamer may be defined as an oligonucleotide or peptide configured to bind the analyte. Such an aptamer may, for example, be configured to interact with, for example bind, various analyte types, such as small molecules, for example amino acids or amines, proteins, metal ions, and microorganisms.

In some embodiments, the capture species and/or analyte may comprise an amplifying element responsive to force, such as the electric and/or magnetic fields, where used, such as electro-active element or moiety or a magnetic-active element or moiety. The amplifying element can increase the response of the capture species to the force used to manipulate the analyte and provide useful kinetic data. This can also enable manipulation where the capture species and/or analyte would otherwise not permit manipulation (e.g. due to no charge). Alternatively or additional, the amplifying element can increase the influence on the measurement signal, for example due to proximity to or location relative to the sensing element. Where the system or method uses amperometric sensing of the analyte, this may comprise electron transfer between the amplifier element becoming faster as the element moves closer to the sensing element. Where, for example, permittivity is measured, this may result from the amplifier element causing a change in permittivity of the sensing element. Where the analyte comprises an amplifying element, this may be an element (e.g. a tag) mixed with the analyte prior to provision to the sensing element, once provided to the sensing element and/or once specifically-bound to the capture species. In some embodiments, the amplifying element may comprise a polymer or a metal, such as a metallic bead (such as a magnetic bead).

One commonly used assay type is enzyme-linked immunosorbent assay (ELISA). ELISA is used for quantifying analytes such as peptides, proteins, antibodies, and hormones. These use a recognition element for selectively (specifically) interacting with, for example binding, the analyte of interest. The recognition element is immobilized on a suitable support. For example, an antigen is immobilized on the support and then complexed with an antibody that is linked to an enzyme. In biosensors and assays, such as ELISA, the non-specific binding of analyte and non-analyte species to the sensing element can result in low signal-to-noise ratio or high limits of detection (e.g. due to non-specific binding). Particularly in biological assays such as ELISA, the size of the species within a sample which non-specifically bind to the surface can also interfere with specific binding of the analyte to the capture species, slowing down the measurement process or even causing the sensor assembly to fail.

In one embodiment, the method further comprises providing a detection species to the sample matrix, the detection species specifically-binding to the analyte; and wherein strength of the electric field configured to remove non-specifically-bound analyte and/or non-analyte species from at least a portion of the sensing element is less than that required to detach specifically-bound detection species from the analyte. The detection species may be provided when the analyte is specifically bound to the capture species or before this occurs. The detection species may be an amplification species or may be a different species, for example provided with the purpose of increasing the selectivity of the measurement. This can be a sandwich assay. In some embodiments, the method is a sandwich ELISA method of detecting a property of an analyte, with the capture species being a capture antibody, the detection species being a detection antibody which may comprise double stranded DNA provided thereon. The DNA may be bound to the detection antibody via a linker, such as streptavidin and biotin. Use of the double stranded DNA in such a system is particularly useful as it is negatively charged and hence is susceptible to manipulation in an electric field.

The detection species can also be used as a means to providing the signal. Although in some embodiments movement of the specifically-bound analyte can be achieved using a flexible capture species, additionally or alternatively, the detection species may provide a flexible or additional flexible component which can be used achieve a response under the manipulation force. For example, in some embodiments, the analyte may be bound to the surface of capture species with the detection species bound thereto, and the detection species may be moved so as to interact with structures such as a through hole. Moreover, the detection species in some embodiments may be detached from the analyte as part of the manipulation or detachment steps, which can provide further information.

In embodiments, the sensing element (e.g. the sensing layer) may comprise analyte interaction portions defined by capture species provided adjacent or on at least one surface of the sensing element. Where structures are provided, this may be adjacent (e.g. next to or abutting) or on the structures. For example, where the structures are recesses or through holes, this may be adjacent or in the recesses or through holes. In some embodiments, the sensing element is functionalized with the capture species. Such functionalization can be achieved in any suitable manner, such as by covalently or non-covalently immobilizing the capture species to the surface. For example, thiol-terminated capture species, such as a thiol-terminated aptamer, can be immobilized, for example grafted, onto the surface of a noble metal, for example gold, electrode.

More generally, the sensing element is arranged to receive a sample matrix. Sample matrix refers to the sample as a whole, including the analyte if present. Thus, it may comprise a carrier (such as a liquid) and the analyte. The sample matrix may be, for example, blood, urine, sweat, tears, etc., and may (potentially) contain the analyte. In an embodiment, the sample matrix is a liquid.

### System, Sensor Assembly and Transduction

In one aspect, there is a system for determining a property of an analyte in a sample, the sample comprising the analyte, non-analyte species and a sample matrix in which the analyte and non-analyte species are contained, the system comprising:
a sensor assembly comprising a sensing element, the sensing element comprising a capture species configured to specifically bind with the analyte and the sensing element providing a measurement signal indicative of the interaction of the sensing element with the sample;
a sample manipulation device for manipulating analyte and/or non-analyte species on the sensing element, the sample manipulation device comprising a plurality of electrodes configured to apply an electric field about the sensing element;
a control unit configured to operate the sample manipulation device; and
a property determination unit configured to determine the property of the analyte;
wherein the control unit is configured to operate the sample manipulation device to generate the electric field so as remove any non-specifically-bound analyte and/or non-analyte species but retain specifically-bound analyte on the sensing element;
wherein the property determination unit is configured to determine a sample baseline measurement based on the measurement signal before and/or during operation of the sample manipulation device; and
wherein the property determination unit is configured to determine the property of the analyte based on the measurement signal after operation of the sample manipulation device.

The system may be configured to perform any of the method steps disclosed herein. Moreover, any of the embodiments set out herein with respect to the method apply equally to the system, and any of the embodiments set out herein with respect to the system apply equally to the method.

For the reasons set out in respect of the method, such a system can be advantageously used to provide an improved analyte measurement. The control unit is configured to operate the sample manipulation device to generate the electric field so as remove any non-specifically-bound analyte and/or non-analyte species but retain specifically-bound analyte on the sensing element, and thus applies the electric field to sample matrix provided on the sensing element, the electric field having a strength less than that required to detach specifically-bound analytes from the sensing element.

In one embodiment, the system may further comprise a signal processing unit configured to process measurement signals received from the sensor assembly; and the property determination unit may receive processed signals and determined the property based on the processed signals. The property determination unit may, in certain embodiments, be configured to determine the property based on (at least) the absolute change in measurement signal and/or the rate of change of the signals.

The control unit, property determination unit and/or signal processing unit may each (individually or combined) be a processor or controller. The control unit may incorporate the property determination unit and/or the signal processing unit or may be in addition to one or both of these. The control unit, signal processing unit, and the property determination unit may be implemented in any suitable manner, with software and/or hardware, to perform the various functions required. One or all of the units may, for example, employ one or more microprocessors programmed using software (for example, microcode) to perform the required functions. Examples of processor components that may be employed in various embodiments include, but are not limited to, conventional microprocessors, application specific integrated circuits (ASICs), and field-programmable gate arrays (FPGAs).

In various implementations, the control unit, the signal processing unit, and/or property determination unit may be associated with one or more non-transitory storage media such as volatile and non-volatile computer memory such as RAM, PROM, EPROM, and EEPROM. The non-transitory storage media may be encoded with one or more programs that, when executed on one or more processors and/or controllers, perform the required functions. Various storage media may be fixed within a processor or controller or may be transportable, such that the one or more programs stored thereon can be loaded into the signal processing unit, property determination unit and/or control unit.

In certain embodiments, the control unit may be configured to alter at least one parameter of the electric field during the step of removing at least a portion of the non-specifically-bound species from the sensing element based on the sample baseline measurement. This can be carried out according to the method set out herein.

In embodiments, the control unit may alternatively or additionally be configured to determine an adjustment factor based on the measurement signal during or after application of the electric field during the step of removing at least a portion of the non-specifically-bound species from the sensing element and after the sample baseline measurement. The control unit may further be configured to adjust the at least one parameter of the application of the electric field based on the adjustment factor. The measurement signal may be pre-processed by the property determination unit, if separate, before being used to determine the adjustment factor. The adjustment factor may be calculated as set out in respect of the method.

In embodiments, the control unit may alternatively or additionally be configured to vary the strength of the electric field during the step of removing at least a portion of the non-specifically-bound components from the sensing element. This can be carried out according to the method set out herein.

In certain embodiments, the control unit is further configured to operate the sample manipulation device so as to apply a force to the sample matrix sufficient to move specifically-bound analyte on the sensing element and thereby manipulate at least a portion of specifically-bound analyte on the sensing element; and wherein the property determination unit is configured to determine the property of the analyte in the sample further based on the measurement signal during and/or after the control unit operates the sample manipulation device to manipulate specifically-bound analyte. This can be after the removal step.

In some embodiments, alternatively or additionally, the control unit is further configured to operate the sample manipulation device so as to apply a force to the sample matrix sufficient to detach specifically-bound analyte on the sensing element; and wherein the property determination unit is configured to determine the property of the analyte in the sample further based on the measurement signal during and/or after the control unit operates the sample manipulation device to detach specifically-bound analyte. This can be after the removal step.

In a further embodiment of the above embodiments, the sample manipulation device generates an electric field and/or a magnetic field to provide the force (which may include an electromagnetic field). The electric and/or magnetic fields can, in embodiments, have any of the properties or parameters set out in respect of the corresponding method.

In the methods and system disclosed herein, the transducer mechanism(s) used to determine the measurement signal may be any suitable method of transduction as this is independent of the manipulation and removal processes. For example, this may comprise measuring the potential (e.g. voltage), current, permittivity, charge and/or frequency.

In some embodiments, the method further comprises modulation of the measurement signal in time, frequency, current, and/or voltage domains. This will generate more signal on biorecognition to differentiate between specific and non-specific binding.

In some embodiments, the property determination is based on a multi-domain measurement, for example, selected from at least two of potential (e.g. voltage), current, charge, permittivity, and/or frequency. This may be that the measurement signal relates to multiple domains.

In some embodiments, the measurement signal is indicative of an impedimetric property of the sensing element or layer and the configuration of the sensing element or layer and the capture species is such that analyte that is specifically bound to the capture species is able to modify an impedimetric property of the sensing element. The impedimetric property of the sensing element or layer (and in some embodiments other components of the sensing element, such as electrodes) is determined by the material properties of the sensing element or layer and, additionally, any component present in the structures. Prior to interaction of the sensing element or layer with the sample, the impedimetric property of the sensing element or layer is determined by the sensing element or layer and any fluid within the structures. However, once a species is received thereon (e.g. around and/or within the structures), the species will cause a change in this impedimetric property. Depending on the analyte property and its modulation of the signal the property to be measured may be due to double layer change or interruption, sensing layer thickness increase, faradaic or non-faradaic processes, transfer of charge, charge storage, or charge induction. For example, when an analyte is bound to the capture species and can interact with the sensing element or layer (or where present structures thereon, such as enter and traverse through a through hole) the analyte will contribute to the impedimetric property of the sensing element or layer. Analytes tend to influence the impedimetric property by a significant margin as compared to a liquid (e.g. a sample matrix) and as such can create a response that is detectable by the system. For example, biomolecules have a permittivity of ~3 compared to a liquid, such as an aqueous solution, which typically have a permittivity of ~80. The response is also one that is directly related to the concentration of analyte (i.e. number of through holes filled and/or to the degree that they are filled), such that this can be used as a qualitative measurement.

In embodiments, the impedimetric property may be selected from a dielectric property (e.g. permittivity), resistance, capacitance, impedance, conductance, or a combination thereof. Depending on the analyte property and its modulation of the signal the property to be measured may be due to double layer change or interruption, sensing layer thickness increase, faradaic or non-faradaic processes, transfer of charge, charge storage, or charge induction. That is, the impedance gives an indication of the opposition to current flow, and this can be influenced by any one of these factors (amongst others). The interaction of an analyte (and the capture species) with the structure will impact these features and therefore impact the impedimetric property. The changes which occur in the sensing element or layer, particularly the sensing element or layer, are as a result of interaction with (e.g. occlusion of) the structures and modulation of electrical phenomena, such as charge transfer, charge induction or current. The systems and methods can therefore determine capacitance, impedance and/or permittivity such that the measurement signal relates to at least one of these, and use this to determine the property of the analyte.

The measurement can be performed using a combination of alternating current (AC) and direct current (DC) and the measurement can be performed at a single frequency or frequency range. In some embodiments, the measurement can be performed using AC measurements. For example, these can enable spectroscopic analysis (e.g. via electrical impedance spectroscopy (EIS)). Such measurements will typically take place over a predetermined frequency range chosen for the particular sensing layer (where the response is effectively linear) and using AC. Measurement of an impedimetric property, such as permittivity, can advantageously be achieved using AC parameters, rather than the traditional DC (only) measurements often used in electrochemical sensing. Use of these parameters over DC measurements alone can advantageously provide a more detailed picture of the response of the interaction with the analyte, and thus provide more information about the analyte itself.

In one embodiment, the impedimetric property is the impedance. This can be converted from measurements of e.g. resistance (the "real" component of the impedance) and reactance (the "imaginary" component of impedance) to capacitance, resistance, impedance, phase angle, using techniques and calculations known in the art.

In some embodiments, the measurement signal is indicative of a dielectric property of the sensing element or layer and the configuration of the sensing element or layer and the capture species is such that analyte that is specifically bound to the capture species is able to modify a dielectric property (e.g. relative permittivity or dielectric constant (both referred to herein as "permittivity")) of the sensing element or layer. Measurement of a dielectric property, such as permittivity, can advantageously be achieved using alternating current (AC) parameters, rather than the traditional direct current (DC) (only) measurements often used in electrochemical sensing.

In one embodiment, the methods and systems are configured to measure impedance using electrochemical impedance spectroscopy (EIS). The measurement signal can therefore be an AC signal which can be processed to provide data on the analyte. Such a measurement can be limited to small potentials thereby limiting the excitation frequencies where a response is essentially linear, and thus providing a more accurate measurement than can be achieved using DC measurements alone. For example, DC in liquid is limited to a voltage below the water (or carrier) electrolysis voltage. In such an embodiment, this can be achieved by addressing the sensing element, and specifically an electrode of the sensing element, using an AC.

In some non-limiting examples, the system includes a user interface, such as a display, for communicating the analyte property determined by the property determination unit. Alternatively or additionally, the system may include a communications interface device, such as a wireless transmitter, configured to transmit the analyte concentration determined by the property determination unit to an external device, such as a personal computer, tablet, smartphone, remote server, etc.

In one aspect, there is provided a computer program comprising computer program code which is configured, when said computer program is run on one or more physical computing devices, to cause said one or more physical computing devices to implement the methods disclosed herein.

In one aspect, there is provided one or more non-transitory computer readable media having a computer program stored thereon, the computer program comprising computer program code which is configured, when said computer program is run on one or more physical computing devices, to cause said one or more physical computing devices to implement the method disclosed herein.

### Sensing element

The sensing element (or "sensing device") provides the measurement signal and therefore may be addressable to provide the measurement signal. For example, the sensing element may comprise a sensing layer and may further comprise an electrode, which electrode may be addressable to provide the measurement signal. Where there are plural electrodes, each may be individually addressable. The electrode(s) may be formed by an electrode layer provided in contact with (on or abutting) the sensing layer, and in some embodiments may be embedded within the sensing layer.

### Sensing Element - Electrode

In some embodiments, the sensing element comprises at least one working electrode. Changes in or interactions with the functional layer formed on the surface can be detected through changes in potential. In other embodiments, the sensor assembly may comprise a sensing element through which current is passed (for example, where the sensing element is a resistive element located between first and second electrodes). In some embodiments, there may be plural electrodes.

In some embodiments, the sensing element comprises an interdigitated electrode (IDE). This can comprise a first electrode and a second electrode, the first and second electrodes arranged in an interdigitated configuration and at least a part of the sensing layer is arranged at least in part between the first electrode and second electrode of the IDE. The IDE is therefore provided with at least a part of the sensing layer between the interdigitated portions of electrodes of the IDE, and therefore at least some of the sensing element, and where present, the structures are located between the interdigitated portions of the electrodes. This provides enables the miniaturisation of the system by eliminating the need for a reference electrode, without sacrificing or reducing the accuracy of the system, enabling use in point-of-care situations.

By interdigitated configuration, it is meant that at the first electrode and second electrode are interdigitated with at least one of the electrodes surrounding at least a part of the other. In some embodiments the first electrode may comprise or be defined by plural extension portions (e.g. fingers) which are interlaced or interlocked with at least one, but optionally plural, extension portions (e.g. fingers) of the second electrode. For example, the first electrode may comprise at least first and second extension portions which are spaced apart from one another to form a recess therebetween and the second electrode comprises at least one first extension portion which is received in the recess. In embodiments, example IDE configurations may include interdigitated comb, spiral or serpentine configurations.

The spacing of the electrodes of the IDE can impact sensitivity. The first and second electrodes in the interdigitated configuration are spaced apart from one another by a spacing of from 10 nm 10 µm, such as from 100 nm to 10 µm, from 500 nm to 10 µm, or from 100 nm to 5 µm. This may be that the extension portions are spaced apart by (e.g. the recess has a width of) 10 nm to 10 µm (such as from 100 nm to 10 µm, from 500 nm to 10 µm, or from 100 nm to 5 µm), with the sensing layer provided therebetween. This spacing is thought to provide an optimal sensitivity, at least because the change in the material properties of the sensing layer will have a significant impact on measurement signal at this scale.

In embodiments, the IDE is at least partially embedded within the sensing layer. For example, the sensing layer may comprise a layer surrounding at least a portion of the electrodes (or extension portions of the IDE). The sensing layer and structures, where present, may extend between the extension portions of the electrodes of the IDE.

In embodiments, the electrodes disclosed herein (e.g. the IDE, such as the first and second electrode of the IDE) may be formed from or comprise metals, metal oxides, metal nitrides, carbon-based materials, a conductive polymer, doped silicon or polysilicon or combinations thereof. In an embodiment, the electrodes may be formed from or comprise gold, silver, copper, platinum, nickel, titanium, titanium nitride, ruthenium, ruthenium oxide or combinations thereof.

### Sensing element - sensing layer

The sensing element comprises (and in some embodiments is formed of) a sensing layer, with which the analyte interacts and which is addressable to provide the measurement signal. In some embodiments, the sensing layer may comprise or be a dielectric layer. In embodiments, the dielectric layer may comprise or be a polymer layer, a glass layer, a glass-ceramic layer, a ceramic layer, a metal oxide layer, a metal nitride layer. a silicon-based layer or combinations thereof. In certain embodiments, the dielectric layer comprises or is a polyimide, silicon dioxide, or silicon nitride layer. In some embodiments, the sensing layer is a nanoporous membrane, with the nanopores forming the through holes (structures) on the nano-scale (e.g. 1 to 1000nm, or 1 to 100 nm). In one embodiment, the sensing element comprises a sensing layer comprising a dielectric material, such as a nitride layer. The measurement can depend on the permittivity and/or capacitance of the layer. This may then further comprise an electrode layer provided in contact with (e.g. on or abutting) the sensing layer. In one further embodiment, the electrode layer may be embedded within the sensing layer.

In one the sensing element comprises a sensing layer, the sensing layer having a plurality of structures formed therein; and wherein the measurement signal is dependent on the location of analyte relative to the plurality of structures in the sensing layer. In embodiments, the sensing layer and the structures formed therein may have any of the structures or features set out herein in respect of the corresponding method and the method may use any of the embodiments set out in respect of the system, such as the features of the sensing layer.

The sensing layer may be comprised of a single layer of material on or in which the structures are formed. Alternatively, or additionally, this may comprise plural layers in a stacked arrangement with the structures extending from the upper surface of the uppermost layer to the lower surface of the lowermost layer. In some embodiments, the layer may comprise plural coplanar layers, where the layers are adjacent to one another and form through hole(s) or well(s) therebetween (i.e. with the through hole or well extending in the gap between the two layers). An electrode may be formed as part of the sensing layer such that the sensing layer comprises this, in which cases the through holes may also extend through the electrode(s), or the sensing element may comprise this as a separate component.

Such an arrangement has been found to provide a particularly useful tool for determining a property of an analyte, since the relative position of the analyte within or around the structures can have a significant effect on the properties. In a further embodiment, the measurement signal is indicative of an impedimetric property (e.g. dielectric property (e.g. permittivity), resistance, capacitance, impedance, conductance, or a combination thereof) of the sensing layer; and wherein the plurality of structures are arranged on or in the sensing layer such that an analyte received between or within the structures modifies the impedimetric property , and therefore the measurement signal. That is, it measures an impedimetric property, which in one embodiment is based on the permittivity of the sensing layer. This can function by the sensing layer having a first impedimetric property (e.g. permittivity) when in the presence of no sample (defined by the sensing layer and any fluid (e.g. a buffer or air) between the structures), and then receipt of the analyte between or within the structures can have significant impact on the impedimetric property (e.g. permittivity). Moreover, the structures in the sensing layer and electric field used also provide additional synergies. The structures provide electric field confinement which increases the strength of the electric field within and adjacent the structures. This increases the ability of the analyte to interact with the structures, and thus the sensing layer, and allows for low voltage sensor systems to create higher strength electric fields than would otherwise be achieved without these types of structure in the sensing layer.

In some embodiments, the plurality of structures define a series of recesses (e.g. wells) or through holes (e.g. via) in the sensing layer, the recesses or through holes have a largest diameter (at their widest point) less than or equal to 2 µm, for example from 50 nm to 1 µm. In some embodiments, the through holes have a diameter (at their widest point) of less than or equal to 1000 nm, for example less than or equal to 800 nm, less than or equal to 500 nm. This may be from 1 nm to 2000 nm, 1 nm to 1000nm, 1 nm to 800 nm, 1 nm to 500 nm, such as 10 nm to 1000 nm, 10 nm to 800, 10 nm to 500 nm, 50 nm to 2000 nm, 50 nm to 1000 nm, 50 nm to 800, 50 nm to 500 nm, 100 nm to 1000 nm, 100 nm to 800, or 100 nm to 500 nm. In one embodiment, the recesses or through holes have a narrowest diameter of at least 0.5 nm, for example at least 1 nm, or at least 10 nm. Recess or through hole radius will impact the response to an analyte: a larger recess or through hole will have a response which is more dependent on the materials within it. A smaller recess or through hole may be selective to particular analytes. Smaller recesses or holes have also been found to provide a higher electric field (field confinement), increasing sensitivity.

In some embodiments, the depth of the recess or through hole may be less than or equal to 2 µm, for example less than or equal to 1000 nm, for example less than or equal to 800 nm, less than or equal to 500 nm. In one embodiment, the one or more through holes have a depth of from 100 nm to 2 µm. This may be 1 nm to 1000 nm, 1 nm to 800 nm, 1 nm to 500 nm, such as 10 nm to 1000 nm, 10 nm to 800, 10 nm to 500 nm, 50 nm to 1000 nm, 50 nm to 800, 50 nm to 500 nm, 100 nm to 1000 nm, 100 nm to 800, or 100 nm to 500 nm. Exemplary combinations include a diameter of 1 nm to 1000 nm and a depth of 1 nm to 1000 nm, such as 10 nm to 500 nm and 10 nm to 500 nm depth. In one embodiment, the depth of the recesses or through holes is from 0.15 µm to 1 µm. Across these depths, noticeable changes in electric field strength and responses have been found, without causing significant variation of electric field across the sensing layer. The depth can be measured from the upper surface of the sensing layer to the lower surface of the sensing layer.

In one embodiment, the sensing layer comprises a plurality of recesses or through holes, and wherein the recesses or through holes are spaced apart by at least 200 nm, at least 500 nm, at least 1000 nm, or at least 2 µm. In some embodiments, this may be from 200 nm to 30 µm, for example from 1 µm to 20 µm, from 2 µm to 20 µm or from 4 µm to 20 µm. These ranges have been found to provide a useful range in which the electric field can vary without impacting performance. It has been found in particular that increasing the recesses or through hole spacing from ~50nm increases the electric field, thus increasing specificity, without reducing molecular transport efficiency. This increase is particularly fast in the range up to 5 µm, after which it levels off. The higher field provided is beneficial but with there are diminishing returns as transport becomes less efficient. Accordingly, the ranges recited herein are particularly effective.

At this size, and particularly the diameter, the analyte can have a significant effect on the property of the sensing layer, such as permittivity. Moving analyte in and out of the structures will therefore provide a significant response, thereby providing additional information as compared to simply receiving analyte on a surface. Movement within and around these structures can be facilitated by the capture species, which may be bound to the sensing element (in some embodiments, the sensing layer) adjacent (i.e. next to or abutting) the structures or on or in the structures, depending on the form of the structures. The recesses may be formed in the layer itself, or may be defined between structures extending above the surface (such as pillars or ridges).

In some embodiments, the sensing layer has been 1 and 1000 through holes, such as 1 to 500 through holes, such as 1 to 20, 1 to 100, 1 to 500, 2 to 20, 2 to 100 or 2 to 500 through holes.

In embodiments, the sensing layer has a thickness of less than or equal to 1000 nm, for example less than or equal to 800 nm, less than or equal to 500 nm. This may be 1 nm to 1000 nm, 1 nm to 800 nm, 1 nm to 500 nm, such as 10 nm to 1000 nm, 10 nm to 800, 10 nm to 500 nm, 50 nm to 1000 nm, 50 nm to 800, 50 nm to 500 nm, 100 nm to 1000 nm, 100 nm to 800, or 100 nm to 500 nm. Accordingly, in some embodiments where the through holes extend through the sensing layer (only), the through holes have a corresponding depth. In other embodiments, the depth of the through hole may be defined by plural layers, such as an electrode, such as the sensing element as a whole.

The density (e.g. the number per unit area) of recesses or through holes present in the sensing layer will impact the response to the present of an analyte. In some embodiments, the through holes may define at least 2% of the surface area of each the upper surface and/or lower surface of the sensing layer contributing to the measurement signal, for example at least 5%, at least 10%, at least 20%. A maximum surface area may be 40%. By contributing to, this may in embodiments refer to the sensing layer located between (e.g. directly between) electrodes used to determine the measurement signal. Measurement may be carried out using SEM or TEM, for example.

In one embodiment, the sensing layer comprises a plurality of recesses or through holes having a largest diameter of less than or equal to 1000 nm, such as from 100 nm to 1000nm, a depth of from 100 nm to 2 µm, such as 100nm to 1 µm, and wherein the recesses or through holes are spaced apart by a spacing of from 1 µm to 20 µm, such as 2 µm to 10 µm.

The through holes or recesses may have any shape. For example, the recesses or through holes may have a cylindrical shape (e.g. with substantially circular or circular openings) or may take the form of tapered openings, channels or slits.

In one specific embodiment of these structures, the plurality of structures comprise a plurality of recesses or wells formed in the sensing layer and/or a plurality of through holes extending from a first surface of the sensing layer to a second surface of the sensing layer. These may have the dimensions mentioned above. Wells (e.g. recesses or grooves in the sensing layer) are advantageously straightforward to manufacture in these surfaces and can provide a useful confinement structure.

Through holes are particularly advantageous as these do not have a truncated structure and therefore the signal can be monitored as it passes through the through hole. Moreover, this can advantageously be used to provide an outlet for removal of components therethrough during the removal step. For example, in some embodiments, the electric field may be applied in a direction that causes movement through the through holes. For example, in one embodiment, wherein a first electrode provided above the sensing layer and a second electrode provided below the sensing layer are configured to provide the electric field. Initial application of the electric field may drive non-specifically-bound components through the through holes. In some cases, this may also simultaneously drive other species in the sample matrix but not yet in contact with or bound to the sensing element towards the sensing element, thereby speeding up binding. Passage through the through hole may provide further information on the species in the sample matrix. In some embodiments, the sample manipulation device is configured to cause specifically-bound analyte to detach and pass through the through holes of the sensing layer.

As noted above, in some embodiments, the sensor assembly comprises the sensing layer comprising a dielectric material and an electrode layer provided in contact with (on or abutting) the sensing layer. In further embodiments, the electrode layer may further comprise at least a plurality of the structures defined herein. For example, the electrode layer may also comprise a plurality of recesses or through holes. These may have the same or different properties to the sensing layer. The features set out herein for the structures of the sensing layer, such as structure and dimensions, apply equally to the electrode layer.

### Temperature control

In some embodiments, the methods, sensor assembly and systems disclosed herein may be configured so as to increase the temperature of the sample, at least in the region of the sensing element. In this way, the energy required to remove the species bound (either specifically or non-specifically) to the capture species and/or sensing element is reduced. Correspondingly, the voltage applied to remove at least a portion of the non-specifically bound or, in some embodiments, the specifically bound species can be reduced. This can allow for small voltages to be applied (enabling miniaturization, for example) and may reduce the likelihood of electrolysis of water. This may also enable further distinction of various binding energies. Accordingly, in embodiments of the method, the method may further comprise increasing the temperature of the sample in a region adjacent to and on the sensing element. This may comprise applying thermal energy to the sample in a region to and on the sensing element. The sensor assembly and systems may further comprise a heater configured such that it can heat a sample provided on the sensing element. In embodiments of the system, the control unit may be configured to increase the temperature of the sample in a region adjacent to and on the sensing element. For example, the control unit may be configured to control the heater. The temperature may be increased by at least 0.5 °C, at least 1 °C, or at least 3 °C. The temperature may be increased to a temperature of at least 22 °C, at least 25 °C or at least 30 °C. In some embodiments, the temperature may be increased to 22 to 35 °C, such as 25 to 35 °C. The temperature may be held at a fixed temperature for the duration of the measurement and binding/debinding steps or may be increased during the debinding step(s) only, for example.

### Specific Uses

In embodiments of the methods, sensor assembly and systems disclosed herein, these can be used to distinguish between minor mutations or modifications of analyte species, such as biomolecules or structures. Specifically, there is a need to be able to distinguish between the types of modifications or differences which occur, either through modification or during synthesis, in species such as DNA or proteins. Traditional methods such as chromatography or mass spectroscopy focus on the mass and/or size of these species, making it different to distinguish where structural differences exist but where the mass or size differences are too small to reliably determine or filter out mismatches. This is particularly the case for biomolecules, where the relative size or mass of the total molecule is typically large as compared to the difference in physical size or mass resulting from the mismatch.

For example, in one embodiment of the methods, sensor assembly and systems disclosed herein, these can be used to identify DNA or RNA mismatches or differentiate/separate DNA or RNA based on the degree of differentiation. DNA or RNA mismatches can occur during the synthesis (e.g. by replication) of DNA or RNA where there is a mismatch between the original DNA or RNA and the synthesized version. This can be as a result of inserting an incorrect nt (or base pair) or deletion of a base pair, for example. Another example is a modification, for example as a result of glycosylation of the DNA/RNA. Such mismatches will alter the binding affinity between the capture species and the DNA or RNA (i.e. analyte) of interest due to a mismatch between the capture species and the analyte. A complete match will have a first binding affinity, some minor mismatch may still bind but with a lower affinity, and more mismatched bases will lead to no selective binding of the capture species and the DNA/RNA. Distinction between the binding components can be determined by the binding affinity, and specifically by the force required to remove the DNA/RNA being analyzed.

For example, in one embodiment, the method may comprise applying a force of a first magnitude which is less than the force used in the detachment step (but may be greater than that in the removal step, since the binding affinity will be higher than for non-specifically bound species). This can be less than the force required to overcome the binding affinity between a non-mismatched (i.e. perfect matched DNA or RNA structure), or in some embodiments a mismatched DNA or RNA structure which is a sufficiently close match (e.g. <1% of bases mismatched, or less than 0.1% of bases) such that only components which are (i) either not specifically bound or (ii) are bound to the capture species but with a binding affinity which is lower than the desired DNA or RNA structure will be removed. This allows the method to strip away any mismatched DNA or RNA. Any DNA or RNA having the desired structure will remain bound to the capture species and be retained on the sensor assembly. Subsequent analysis can be used to determine the amount of DNA/RNA which remains, for example measurement, manipulation and/or detachment steps.

Additionally or alternatively, this can be used as a filter. Specifically, the non-desired species can be removed from the system after it has been detached from the capture species. Subsequently, the disclosed detachment steps can be used to detach the desired DNA or RNA from the sensor assembly and retained as a separate component.

In one other embodiment, and using the same techniques and steps as set out above for the DNA/RNA mismatch, another embodiment includes determining changes in a protein, for example due to glycosylation, or filtering out glycosylated and non-glycosylated proteins.

### Specific Embodiments

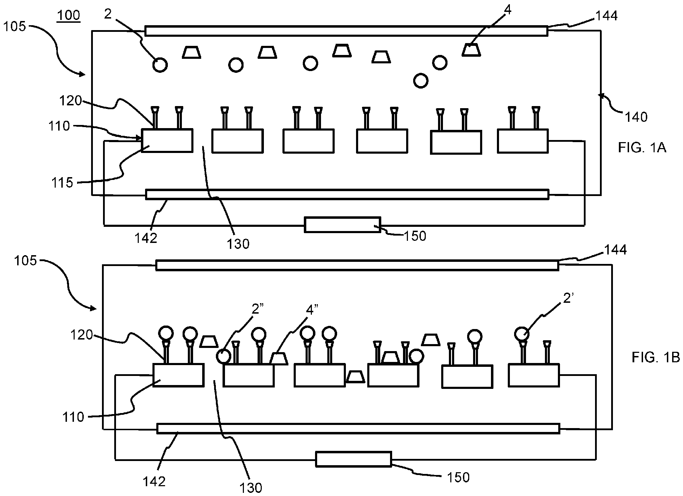

Figs. 1A to 1E schematically depict a cross-section of a system 100 for determining a property of an analyte 2 in a sample, the sample comprising the analyte 2, non-analyte species 4 and a sample matrix in which the analyte 2 and non-analyte species 4 are contained. It will be appreciated that the schematic drawings are provided to exemplify the concepts provided herein and the components, such as the through holes and capture species, are not intended to be to scale.

The system 100 comprises a sensor assembly 105 comprising a sensing element 110, the sensing element 110 comprising a sensing layer 115 having a plurality of through holes 130 extending from an upper surface to a lower surface. The sensing layer 115 is suspended in a chamber such that fluid resides on both sides of the sensing layer 115 and with the through holes 130 providing for passage of fluid from the region above the sensing layer 115 to below the sensing layer. The sensing layer 115 is functionalised with capture species 120 configured to specifically bind with the analyte provided on the upper surface adjacent the through holes 130. The sensing layer 115 and capture species 120 are selected and configured so that the sensing element 110 can provide a measurement signal indicative of the interaction of the sample with the sensing element 110.

The system 100 also comprises a sample manipulation device 140 which is for manipulating analyte 2 and/or non-analyte species 4 in the sample matrix (e.g. on the sensing element 110). The sample manipulation device 140 comprises a first electrode 144 provided above the sensing layer 115 and a second electrode 142 electrically connected to the first electrode 144 and located below the sensing layer 115. The first electrode 144 and second electrode 142 are configured to apply an electric field through the sample matrix and about the sensing element 110 so that movement of the analyte 2 and non-analyte species 4 within the sample matrix can be controlled by the electric field generated therebetween. Specifically, the arrangement of the first and second electrodes 144, 142 above and below the sensing layer 115 permits movement substantially perpendicular to the upper and lower surfaces of the (planar) sensing layer 115 and in a direction through the through holes 130. This enables movement from one side of the sensing layer 115 to the other side, for example.

The system 100 further comprises a control unit 150 which is a processor configured to operate the sample manipulation device 140. Specifically, the control unit 150 is configured to operate the sample manipulation device 140 to generate the electric field using the first and second electrodes 144, 142, as will be explained in more detail below. In this embodiment, the control unit 150 also comprises a property determination unit configured to determine the property of the analyte. This determination is based on the measurement signal obtained from the sensing element 110, as detailed below.

In use, analyte 2 provided to the system 100 will specifically bind to the capture species to form specifically-bound analyte 2', as depicted in Fig. 1B. Additionally, some analyte may non-specifically bind to the surface of the sensing layer 115 to form non-specifically-bound analyte 2". Non-analyte species 4 may also non-specifically bind to sensing layer 115 to form further non-specifically-bound non-analyte species 4". Although the main interaction of interest is that between the capture species 120 and the analyte 2, which will contribute to a response in the measurement signal, the non-specific binding of the species will also contribute to the signal. This interaction can reduce the sensitivity of the measurement and slow down the measurement process. In some cases, non-specific binding can lead to sensor failure.

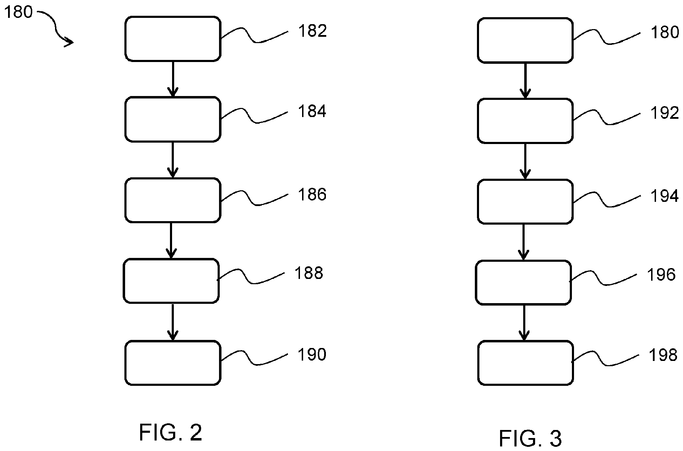

A method 180 of determining a property of an analyte in a sample, using the system 100, is depicted in Fig. 2 and set out below with reference to Figs. 1A to 1C. The method 180 comprises providing 182 the sensor assembly 105 followed by providing 184 the sample to the sensing element 110. As shown in Fig. 1B, at least a portion of analyte 2 and non-analyte species 4 in the sample non-specifically bind to the sensing layer 115 to form non-specifically-bound analyte 2" and non-specifically-bound non-analyte species 4" and least a portion of analyte 2 specifically-binds to the sensing layer 115 to form specifically-bound analyte 2'.

The method 180 further comprises obtaining a sample baseline measurement 186 based on the measurement signal. The measurement signal can be taken at the point in time depicted by Fig. 1B and therefore is based on the contribution to the signal provided by specifically-bound analyte 2' and the non-specifically-bound analyte 2" and non-specifically-bound non-analyte 4". This measurement signal is obtained and processed by the control unit 150.

The method 180 further comprises removing at least a portion of the non-specifically-bound analyte and/or non-analyte species from the sensing element by applying an electric field to the sample matrix ("removal step" 188). The electric field in system 100 is formed using the sample manipulation device 140 under the action of the control unit 150.

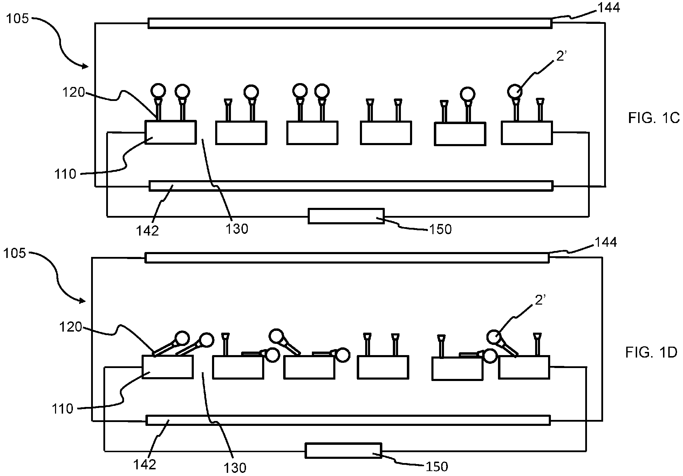

This removal step 188 causes removal of non-specifically-bound analyte and/or non-analyte species from at least a portion of the sensing element by providing a force which acts on the species driving them away from one of the first and second electrodes 144, 142 and towards the other. In one implementation depicted in Fig. 1C, the electric field is configured to move the species towards the second electrode 142, and as such the species move from the region above the upper surface of the sensing layer 115 to the region below the lower surface of the sensing layer 115. This can also lead to passage through the through holes 130. During this step, the electric field is applied with a strength that is less than that required to detach specifically-bound analyte 2' from the sensing layer 115. This can be determined based on strength of the interaction (affinity) between the capture species 120 and the analyte 2.

The removal step 188 accordingly strips away the non-specifically-bound species 2", 4" from the sensing layer 115 leaving behind specifically-bound analyte 2' on the surface. In this way, the contribution of the non-specifically-bound species to the measurement signal is eliminated or at least reduced, thereby improving the accuracy of the measurement overall.

The method can therefore further comprise determining the property of the analyte in the sample 190 based on the measurement signal after the removal step 188.

In such a system 100 and method 180, determination of the sample baseline measurement 186 can be particularly useful. The measurement itself can be used to provide information on non-specific binding in the system, for example the speed of the incubation process and the degree of binding. Moreover, it can further be used to improve the accuracy of subsequent measurement processes.

For example, in some embodiments of the method 180, the sample baseline measurement is used to determine at least one parameter of the removal step 188. This can be prior to the beginning of the removal step 188 or during the removal step 188. For example, the level of the sample baseline measurement may determine at least one of the duration of the removal step or the parameters of the electric field, such as at least one of the duration of the application of the electric field to the sample matrix, the strength of the electric field, or the number of repetitions of the application of the electric field. This may be a feedback loop whereby the control unit 150 may further determine an adjustment factor based on the measurement signal during or after application of the electric field and during the removal step 188 but after the initial sample baseline measurement. This essentially takes an intermediate measurement during the removal step 188 and the adjustment parameter may be used to change one of the parameters of the removal step 188. This can be, for example, an increase in the duration and/or intensity if the response to the removal step 188 is having little or no effect or a decrease in the duration and/or intensity if it is thought that the removal step 188 has finished.

One useful metric for monitoring the removal step 188, for example particularly where the magnitude of non-specific binding is not known, can be rate of change. The adjustment factor can therefore be based on the rate of change in the measurement signal during the removal step 188.

In other embodiments, which provide an additional or alternative use of the sample baseline measurement, the sample baseline measurement can be used to compensate measurement signals in future measurements and avoid the need for a repeated removal step 188. For example, a signal compensation factor can be determined by the control unit 150, which can be derived from determining the amount of the measurement signal attributable to non-specific binding, and the subsequent determination of the future measurement can use the measurement signal for the second sample and the signal compensation factor.

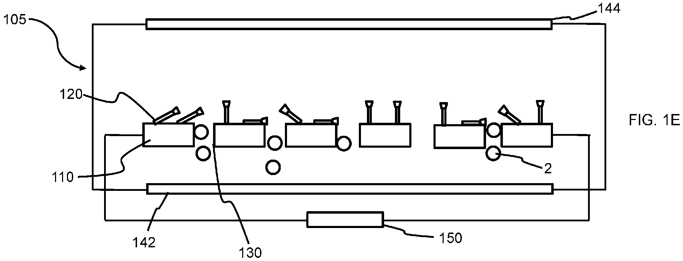

Fig. 3. depicts additional method steps 192-196 which can be carried out in addition to the method depicted in Fig. 2 and in conjunction with system 100. The additional steps depicted therein can follow on from method 180, as shown in Fig. 3 and depicted in Figs. 1D and 1E.

In this embodiment, the method further comprises manipulating at least a portion of the specifically-bound analyte ("manipulation step" 192) after the removal step 188. In this embodiment, the electric field generated between the first and second electrodes 144, 142 has a higher strength than in the removal step 188 so that there is movement of the specifically-bound analyte on the sensing layer 115. With the configuration of the first and second electrodes 144, 142 above and below the sensing layer 115 and the through holes 130 located adjacent the capture species 120, this movement can move the specifically-bound analyte 2' into the through holes 130 and, depending on the length and position of the capture species, in some embodiments through the through holes 130, as depicted in Fig. 1D.

This manipulation step 192 will generate specific responses and change in the response of the sensing element (i.e. the measurement signal). This can be monitored over time to build up an interaction picture and provide further information on the analyte. For example, the kinetics of movement or the specific interactions could be used as a fingerprint indicative of the presence of the analyte and a quantity of the analyte. For example, the response may vary based on the speed of movement or interaction with the through holes 130 based on the size and nature of the analyte 2. Accordingly, the method further comprises determining the property of the analyte 194 further based on the measurement signal during and/or after the manipulation step 192.

In some embodiments, determining the property of the analyte 194 is based on the rate of change in the measurement signal during the step of manipulating the specifically-bound analyte. This can help to build up the picture or fingerprint providing a higher degree of accuracy and certainty. Unlike typical electrochemical measurements, the rate of change is particularly beneficial in these methods given the manipulation of the analyte and therefore the varying response.

The method in this embodiment further comprises detaching at least a portion of the specifically-bound analyte ("detachment step" 196) after removal step 188. Detaching specifically-bound analyte comprises applying a force to the sample matrix sufficient to detach specifically-bound analyte from the sensing element, which in this is achieved by further increasing the strength of the electric field provided by the sample manipulation device 140 to a strength that overcomes the affinity between the capture species 120 and the analyte 2.

Fig. 1E shows the result of the detachment step 196. Due to the specific electric field applied, movement of the analyte 2 from the first electrode 144 to the second electrode 142 occurs such that the analyte passes through the through holes 130 and to the underside of the sensing layer 115. The method accordingly further comprises determining the property of the analyte in the sample 198 based on the measurement signal during and/or after the detachment step 196.

As with the manipulation step 192, the detachment step 196 may be a simple linear application of the force (i.e. the electric field in this embodiment) or it may comprise different applications of force to obtain further information about the response of the sensor assembly 105.

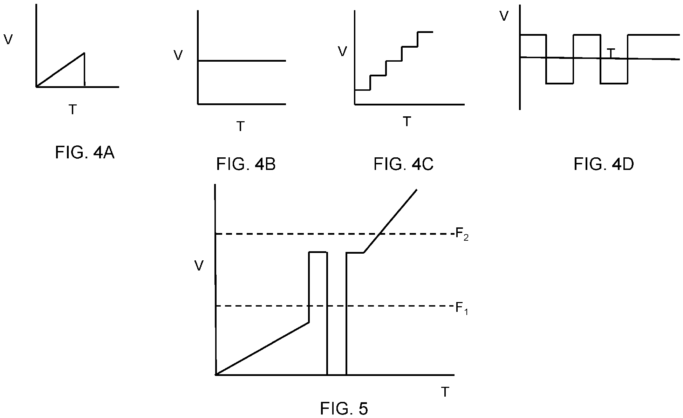

Figs. 4A to 4D depict example parameters for applying the electric field in the removal step 188, with voltage (V) plotted against time (T). The method 180 set above can different parameters during the removal step 188, and additionally any manipulation step 192 and detachment step 196. Fig. 4A depicts a linear ramp over time from no electric field to a maximum (which for the removal step is less than that which would cause detachment of specifically-bound analyte 2'). This can lead to staggered removal of the non-specifically-bound species. Fig. 4B depicts a hold at a single intensity. Fig. 4C depicts a series of increasing steps. This too may lead to staggered removal of the non-specifically-bound species and may provide useful information about particular non-specifically-bound species where the relative non-specifically-bound affinities are known, for example. Fig. 4D depicts an alternating application of the electric field where the polarity is switched. This can be used to drive the non-specifically-bound species back and forth through the through holes 130, for example, and in and out of interaction with the sensing element 110 thereby building up a picture. The final application of the electric field may comprise a hold at one intensity so as to remove the non-specifically-bound species from the sensing element 110.

Although Figs. 4A to 4D depict the profiles that could be used for the removal step 188, it will be appreciated that the same profiles could be used for the manipulation step 192 and detachment step 196 with modification for the maximum or magnitude of the force applied (e.g. the electric field strength).

Fig. 5 depicts an example for applying the electric field in the method 180 and subsequent manipulation step 192 and detachment step 196, with voltage (V) plotted against time (T). This depicts a first linear ramp for a period of time in the removal step 188 where the non-specifically-bound components are removed. The voltage is then increase above F₁, with F₁ corresponding to the force at which there is substantial movement of the specifically-bound analyte 2' on the sensing layer 115 (as depicted in Fig. 1D), where it is held for a period of time before the electric field is turned off. This allows the capture species 120 and specifically-bound analyte 2' to relax, before the electric field is reapplied for a period of time. There is a final ramp to a voltage which provides a force above F₂, with F₂ corresponding to the force required to overcome the binding between capture species 120 and analyte 2.

Although the embodiment of Fig. 5 has been depicted with reference to use of the same electric field throughout each of the removal step 188, the manipulation step 192 and the detachment step 196, it will be appreciated that these may be applied using different manipulation means. For example, the manipulation step 192 and the detachment step 196 may be carried out using a separate manipulation unit, for example a magnetic field generator or a separate electrode array.

Although the system of Figs. 1A has been depicted with a sensing layer having through holes and with the sample manipulation device 140 comprising a first electrode 144 located above the sensing layer 115 and the second electrode 142 located below the sensing layer 115, it will be appreciated that other embodiments are possible. For example, the sensing element may comprise a planar electrode and the electric field may be generated by electrodes which provide movement across the surface of the planar electrode.

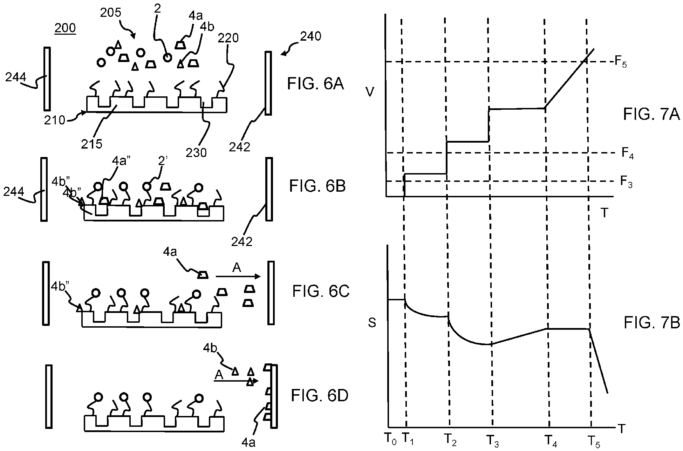

Figs. 6A to 6D schematically depict an embodiment of a system 200 for detecting a property of an analyte, which can be used with the methods disclosed herein, and Figs. 7A and 7B schematically depict the voltage (V) applied to a first electrode 244 and second electrode 242 over time (T) for generating an electric field and a schematic of the measurement signal (S) measured (i.e. the response) over time (T) during the method, respectively. The system 200 is for detecting an analyte 2 in a sample, the sample comprising a sample matrix in which there is analyte 2, a first non-analyte species 4a and a second non-analyte species 4b.

The system 200 comprises a sensor assembly 205 comprising a sensing element 210, the sensing element 210 comprising a sensing layer 215 having a plurality of wells 230 formed therein in an upper surface. The sensing layer 215 received liquid sample on the upper surface of the sensing layer 215. This upper surface of the sensing layer 215 is functionalised with capture species 220 configured to specifically bind with the analyte 2 provided, with the capture species 220 located adjacent the wells 230. The sensing layer 215 and capture species are selected and configured so that the sensing element 210 can provide a measurement signal indicative of the interaction of the sample with the sensing element 210.

The system 200 also comprises a sample manipulation device 240 for manipulating analyte 2 and the first non-analyte species 4a and second non-analyte species 4b in the sample matrix. The sample manipulation device 240 comprises a first electrode 244 provided at one end of the sensing layer 215 and a second electrode 242 electrically connected to the first electrode 244 and at the opposite end of the sensing layer 215. The first electrode 244 and second electrode 242 are arranged and configured so that they form an electric field across the upper surface of the sensing layer 215 which drives the analyte 2, the first non-analyte species 41 and the second non-analyte species 4b in a direction parallel to the sensing layer 215 surface.

The system 200 further comprises a control unit (not shown) which is a processor configured to operate the sample manipulation device 240. In this embodiment, the control unit also comprises a property determination unit (not shown) configured to determine the property of the analyte. This determination is based on the measurement signal obtained from the sensing element 210.

In use, analyte 2 will specifically bind to the capture species 220 to form specifically-bound analyte 2'. At least some of the first and second non-analyte species 4a, 4b may non-specifically bind to sensing layer 215 to form first and second non-specifically-bound species 4a", 4b".

Referring to Figs. 7A and 7B and Fig. 6B, this correlates to point T₀, where it can be seen that, in this embodiment, no voltage is applied to sample manipulation device 240 such that there is no electric field applied. A signal corresponding to the combination of contributions from all of the species specifically and non-specifically-bound on the sensing layer 215 are present. The signal between T₀ to T₁ provides an initial sample baseline measurement.

At time point T₁, the sample manipulation device 240 is activated to carry out a step of removing the first and second non-specifically-bound species 4a", 4b". At first, the voltage is increased to a first level. This first level is greater than the force required to remove the first non-specifically-bound non-analyte species 4a" (Fs) and therefore this first non-specifically-bound species 4a" is detached from the sensing layer 215 and moved in the direction of the second electrode 242. This is depicted in Fig. 6C. As a result, there is a change in the measurement signal between T₁ and T₂ At first this is a steep drop but the rate of change in the measurement signal slows. At this point, non-specifically-bound non-analyte species 4b" is still bound to the surface and the sample baseline measurement may indicate this to user, for example compared to an estimate of the property in the analyte. Alternatively or additionally, if there is still some change visible in the baseline signal, this may prompt the user to alter a parameter of the electric field.

In some embodiments, the degree to which the parameter is altered may be based on the sample baseline signal. In particular, the control unit may further determine an adjustment factor based on the measurement signal during this removal step 188. This essentially takes an intermediate measurement during the removal step 188 and the adjustment parameter may be based on this and used to change one of the parameters of the removal step 188. In other embodiments, this may be based on a predetermined change.

In this embodiment, at time point T₂, the sample manipulation device 240 modifies the voltage to a second level. This second level is greater than the force required to remove the second non-specifically-bound non-analyte species 4b" (F₄) and therefore this second non-specifically-bound non-analyte species 4b" is detached from the sensing layer 215 and moved in the direction of the second electrode 242. As a result, there is a change in the measurement signal between T₂ and T₃ At first this is a steep drop but the rate of change in the measurement signal slows as the amount of the second non-specifically-bound non-analyte species 4b" depletes on the sensing layer 215. This is depicted in Fig. 6D.

At this point, the contribution of the non-specifically binding species to the measurement signal has been eliminated and measurement of the analyte 2 property can begin. For example, at this point, the method may comprise manipulating the specifically-bound analyte 2' on the surface of the sensing layer 215. This can be is depicted in Figs. 7A and 7B between T₃ and T₄. In this embodiment, the increase in response (simplified) results from the specifically-bound analyte 2' moving closer to the surface of the sensing layer 215.

After this step, the voltage of the electric field is then ramped up to cause detachment of the specifically-bound analyte 2' from the sensing. At the point that the force is greater than that between the specifically-bound analyte 2' and the capture species 220 (F₅), there is a change in signal corresponding to the loss of analyte 2 from the surface. The specific response will depend on the sensing element 210 and the arrangement of the electric field, or in embodiments using some other application of force, the direction of that force.

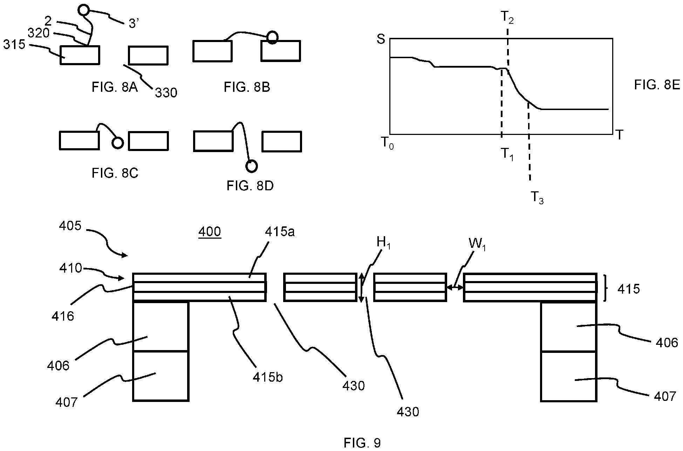

Figs. 8A to 8D provide schematic cross-section views of a sensing layer 315 with a through hole 330 provided therein and the use of an amplification bead 3'. Fig. 8E schematically depicts a graph showing an example signal (S) corresponding to the configurations shown in Figs. 8A to 8D, plotted against time (T). Such a sensing layer 315 and the corresponding method of use can be used in any of the systems and methods disclosed herein.

Specifically, the sensing layer 315 is provided with a capture species 320 to which an analyte 2 has bound. The system has also been provided with an amplification bead 3' which is attached to the analyte 2, in a similar manner to a sandwich assay. In this embodiment, the amplification bead 3' is a magnetic metallic bead and can be manipulated using a magnetic field (not shown) and which, together with the analyte 2, will have an effect on the impedimetric property of the sensing layer 315. Fig 8A. depicts an initial configuration in which there is no magnetic field applied and the signal is at a baseline between T₀ and T₁. As the magnetic field is applied, amplification bead 3' is driven towards the sensing layer 315 (see Fig. 8B) but does not yet enter the through hole 330. This causes a change in signal between T₁ and T₂, as depicted in Fig. 8E. As the magnetic field is further applied, amplification bead 3' is driven through the through hole 330. Fig. 8C depicts the amplification bead within the through hole 330 which causes a large change in the impedimetric property of the sensing layer 315. This in turn causes a large change in signal (e.g. between T₂ and T₃). Finally, as the amplification bead 3' exits the through hole 330 on the other side of the through hole 330, the signal levels out with a new baseline (corresponding to the signal with the analyte 2 through the through hole 330). The rate of change and difference in signals before and after the manipulation process can all provide data used to confirm the presence and amount of an analyte.

Fig. 9 schematically depicts a cross-section through a system 400 comprising a sensor assembly 405. The sensor assembly 405 comprises a sensing element 410, the sensing element 410 comprising a sensing layer 415 formed of a passivation layer 415a providing the upper surface, an IDE 416 provided beneath the passivation layer 415a and a base sensing layer 415b provided beneath the IDE 416. The IDE 416 is accordingly embedded within sensing layer 415. The sensing layer 415 also comprises a plurality of through holes 430 extending from an upper surface of the passivation layer 415a to the lower surface of the base sensing layer 415b. In this embodiment, the sensing element 410 is suspended in a fluid chamber such that fluid resides on both sides of the sensing layer 415 by elevating the sensing element 410 on supports 406, in this case an oxide layer, which oxide layer is formed on silicon 407. Such sensor assemblies 405 can advantageously be formed using traditional CMOS manufacturing techniques.

The passivation layer 415a provides a surface which is more straightforward to functionalise and which can be used to limit interaction of the IDE 416 with species on the upper surface of the sensing layer 415, thereby focusing the signal changes on interaction with the through holes 430.

In one particular example based on the system 400, the system 400 can be used for performing an ELISA assay. The structure used in this particular example can comprise a silicon oxide supports 406 with a thickness of 1-2µm. The passivation layer 415a is a 70nm thick passivation layer. The IDE layer is a 50nm thick titanium nitride (TiN) layer. The base sensing layer 415b is a 100nm silicon nitride (Si₃N₄) dielectric layer. This creates a total depth of through hole 330 through the sensing layer 415 of 220 nm. The through holes 430 have a width of 400 nm. These dimensions provide a through hole 430 which is responsive to ELISA structures. For example, the capture antibody can have a length of 10-15 nm and it may use a detection antibody provided with a double-stranded DNA of ~300 nm long, with a 2nm diameter (900 base pairs).

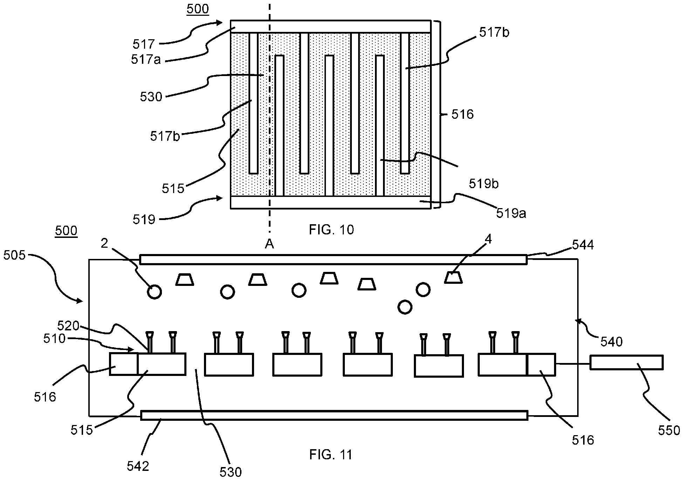

Figs. 10 and 11 schematically depict a system 500 for determining a property of an analyte 2 in a sample. Fig. 10 provides a schematic plan view of the system 500 and Fig. 11 provides a schematic cross-sectional view of the system 500 along line A shown in Fig. 10. The system 500 can be used in conjunction with the methods disclosed herein.

The system 500 is similar to the system 100 depicted in Fig. 1 in that it comprises a sensor assembly 505 comprising a sensing element 510, the sensing element comprising a sensing layer 515 having a plurality of through holes 530 extending from an upper surface to a lower surface. The sensing layer 515 is suspended in a chamber such that fluid resides on both sides of the sensing layer 515 and with the through holes 530 providing for passage of fluid from the region above the sensing layer 515 to below the sensing layer. Sensing layer 515 is functionalised with capture species 520 configured to specifically bind with the analyte provided on the upper surface adjacent the through holes 530.

The sensing element 510 also comprises an interdigitated electrode (IDE) 516 formed from a first electrode 517 and a second electrode 519 arranged in an interdigitated configuration, as visible from the plan view of Fig. 10. Specifically, the first electrode 517 comprises a first connector 517a which extends along one side of the sensing layer 515 and from which plural first extension portions 517b extend perpendicularly. These first extension portions 517b are spaced apart from one another. The second electrode 519 comprises a second connector 519a which extends along the opposite side of the sensing layer 515 to the second connector 519a and plural second extension portions 519b extend perpendicularly from the second connector 519a towards the first connector 517a. The second extension portions 519b are also spaced apart from one another but are staggered as compared to the first extension portions 517b. In this way, the second extension portions 519b are received between the first extension portions 517b to form the IDE 516. This can be referred to as an interdigitated comb configuration.

The sensing layer 515 is arranged so that the majority of the sensing layer is located between the first extension portions 517b and second extension portions 519b of the first electrode 517 and second electrode 519, respectively. The IDE 516 is therefore provided with the through holes 530 of the sensing layer 515 between the interdigitated portions of the first electrode 517 and second electrode 519. This allows the IDE 516 to detect changes in the impedimetric properties of the sensing layer 515 caused by interaction of species with (such as intrusion into) the through holes. Moreover, the use of the IDE 516 in this way enables the miniaturisation of the system by eliminating the need for a reference electrode, without sacrificing or reducing the accuracy of the system, enabling use in point-of-care situations.

As such, the sensing layer 515, capture species 520 and IDE 516 are selected and configured so that the sensing element 510 can provide a measurement signal indicative of the interaction of the sample with the sensing element 510.

As with the earlier systems disclosed herein, system 500 further comprises a sample manipulation device 540 which is for manipulating analyte 2 and/or non-analyte species 4 in the sample matrix (e.g. on the sensing element 510). The sample manipulation device 540 comprises a first manipulation electrode 544 provided above the sensing layer 515 and a second manipulation electrode 542 electrically connected to the first manipulation electrode 544 and located below the sensing layer 515. These are separate to the IDE 516. The system 500 further comprises a control unit 550 which is a processor configured to operate the sample manipulation device 540. Specifically, the control unit 550 is configured to operate the sample manipulation device 540 to generate the electric field using the first manipulation electrode 544 and the second manipulation electrode 542. In this embodiment, the control unit 550 also comprises a property determination unit configured to determine the property of the analyte. This determination is based on the measurement signal obtained from the sensing element 510 (specifically, by addressing the IDE 516).

It should be understood that the detailed description and specific examples, while indicating exemplary embodiments of the apparatus, systems and methods, are intended for purposes of illustration only and are not intended to limit the scope. These and other features, aspects, and advantages of the apparatus, systems and methods of the present invention can be better understood from the description, appended claims or aspects, and accompanying drawings. It should be understood that the Figures are merely schematic and are not drawn to scale. It should also be understood that the same reference numerals are used throughout the figures to indicate the same or similar parts.

Other variations to the disclosed embodiments can be understood and effected by those skilled in the art in practicing the disclosure, from a study of the drawings, the disclosure, and the appended aspects or claims. In the aspects or claims, the word "comprising" does not exclude other elements or steps, and the indefinite article "a" or "an" does not exclude a plurality. The mere fact that certain measures are recited in mutually different dependent aspects or claims does not indicate that a combination of these measures cannot be used to advantage. Any reference signs in the claims should not be construed as limiting the scope.

Aspects of the invention will now be described:
Aspect 1. A method for determining a property of an analyte in a sample, the sample comprising the analyte, non-analyte species and a sample matrix in which the analyte and non-analyte species are contained, the method comprising: providing a sensor assembly, the sensor assembly comprising a sensing element comprising a capture species configured to specifically bind with the analyte, the sensing element providing a measurement signal indicative of the interaction of the sensing element with the sample; providing the sample to the sensing element, wherein at least a portion of analyte and/or non-analyte species in the sample non-specifically bind to the sensing element and wherein least a portion of analyte specifically-binds to the sensing element; obtaining a sample baseline measurement based on the measurement signal; removing at least a portion of the non-specifically-bound analyte and/or non-analyte species from the sensing element by applying an electric field to the sample matrix, the electric field configured to remove non-specifically-bound analyte and/or non-analyte species from at least a portion of the sensing element but having a strength less than that required to detach specifically-bound analyte from the sensing element; and determining the property of the analyte in the sample based on the measurement signal after the step of removing at least a portion of the non-specifically-bound analyte and/or non-analyte species from the sensing element.
Aspect 2. The method of aspect 1, wherein the sample baseline measurement is used to determine at least one parameter of the step of removing at least a portion of the non-specifically-bound analyte and/or non-analyte species from the sensing element.
Aspect 3. The method of either of aspect 1 or aspect 2, further comprising determining an adjustment factor based on the measurement signal during the step of removing at least a portion of the non-specifically-bound analyte and/or non-analyte species from the sensing element and after the sample baseline measurement, and further comprising adjusting at least one parameter of the step of removing at least a portion of the non-specifically-bound analyte and/or non-analyte species from the sensing element based on the adjustment factor.
Aspect 4. The method of aspect 3, wherein the adjustment factor is based on the rate of change in the measurement signal during the step of removing at least a portion of the non-specifically-bound analyte and/or non-analyte components from the sensing element.
Aspect 5. The method of any preceding aspect, further comprising: subsequently providing a second sample to the sensor assembly, the sample comprising analyte, non-analyte species and a sample matrix in which the analyte and non-analyte species are contained; and determining the property of the analyte in the second sample based on the measurement signal for the second sample and a signal compensation factor, wherein the signal compensation factor is based on the sample baseline measurement and the measurement signal for the sample.
Aspect 6. The method of any preceding aspect, wherein the sample baseline measurement is obtained at least during the step of removing at least a portion of the non-specifically-bound analyte and/or non-analyte species from the sensing element, and the sample baseline measurement comprises the rate of change in the measurement signal.
Aspect 7. The method of any preceding aspect, wherein the strength of the electric field is varied during the step of removing at least a portion of the non-specifically-bound analyte and/or non-analyte components from the sensing element.
Aspect 8. The method of any preceding aspect, further comprising manipulating at least a portion of the specifically-bound analyte after the step of removing at least a portion of the non-specifically-bound analyte and/or non-analyte components from the sensing element, wherein manipulating specifically-bound analyte comprises applying a force to the sample matrix sufficient to move specifically-bound analyte on the sensing element; and wherein determining the property of the analyte in the sample is based on the measurement signal during and/or after the step of manipulating specifically-bound analyte.
Aspect 9. The method of aspect 8, wherein determining the property of the analyte is based on the rate of change in the measurement signal during the step of manipulating specifically-bound analyte.
Aspect 10. The method of any preceding aspect, further comprising detaching at least a portion of the specifically-bound analyte after the step of removing at least a portion of the non-specifically-bound analyte and/or non-analyte components from the sensing element, wherein detaching specifically-bound analyte comprises applying a force to the sample matrix sufficient to detach specifically-bound analyte from the sensing element; and wherein determining the property of the analyte in the sample is based on the measurement signal during and/or after the step of detaching the specifically-bound analyte.
Aspect 11. The method of aspect 10, wherein determining the property of the analyte is based on the rate of change in the measurement signal during the step of detaching the specifically-bound analyte.
Aspect 12. The method of aspect 10 or aspect 11, wherein applying a force to the sample matrix sufficient to detach specifically-bound analyte from the sensing element comprises applying a first force and increasing the applied force to a second force, optionally wherein the first force is from 0 to 50 pN and the second force is greater than the first force by at least 20 pN.
Aspect 13. The method of any of aspects 8 to 12, wherein the force is provided by at least one of an electric field or a magnetic field.
Aspect 14. The method of any preceding aspect, wherein the method further comprises providing a detection species to the sample matrix, the detection species specifically-binding to the analyte; and wherein strength of the electric field configured to remove non-specifically-bound analyte and/or non-analyte species from at least a portion of the sensing element is less than that required to detach specifically-bound detection species from the analyte.
Aspect 15. The method of any preceding aspect, wherein the sensing element comprises a sensing layer, the sensing layer having a plurality of structures formed therein; and wherein the measurement signal is dependent on the location of the analyte relative to the plurality of structures in the sensing layer.
Aspect 16. The method of aspect 14, wherein the measurement signal is indicative of an impedimetric property of the sensing layer and the configuration of the sensing layer and the capture species is such that analyte that is specifically bound to the capture species is able to modify an impedimetric property of the sensing layer.
Aspect 17. The method of either of aspect 14 or aspect 15, wherein the plurality of structures comprise a plurality of wells formed in the sensing layer and/or a plurality of through holes extending from a first surface of the sensing layer to a second surface of the sensing layer.
Aspect 18. The method of any of aspects 14 to 16, wherein a first electrode provided above the sensing layer and a second electrode provided below the sensing layer are configured to provide the electric field.
Aspect 19. The method of any preceding aspect, wherein the step of providing the sample matrix to the sensing element comprises: applying an electric field to the sample matrix on the sensing element, the electric field being configured so as to cause movement of the analyte towards the capture species.
Aspect 20. The method of any preceding aspect, wherein applying an electric field to the sample matrix, the electric field configured to remove non-specifically-bound analyte and/or non-analyte species from at least a portion of the sensing element but having a strength less than that required to detach specifically-bound analyte from the sensing element applying the force to cause at least a portion of the specifically-bound analyte species to debind from the capture species comprises: applying a first force and increasing the applied force to a second force, optionally wherein the first force is from 0 to 20 pN and the second force is greater than the first force by at least 20 pN.
Aspect 21. A system for determining a property of an analyte in a sample, the sample comprising the analyte, non-analyte species and a sample matrix in which the analyte and non-analyte species are contained, the system comprising: a sensor assembly comprising a sensing element, the sensing element comprising a capture species configured to specifically bind with the analyte and the sensing element providing a measurement signal indicative of the interaction of the sensing element with the sample; a sample manipulation device for manipulating analyte and/or non-analyte species on the sensing element, the sample manipulation device comprising a plurality of electrodes configured to apply an electric field about the sensing element; a control unit configured to operate the sample manipulation device; and a property determination unit configured to determine the property of the analyte, wherein the control unit is configured to operate the sample manipulation device to generate the electric field so as remove any non-specifically-bound analyte and/or non-analyte species but retain specifically-bound analyte on the sensing element; wherein the property determination unit is configured to determine a sample baseline measurement based on the measurement signal before and/or during operation of the sample manipulation device; and wherein the property determination unit is configured to determine the property of the analyte based on the measurement signal after operation of the sample manipulation device.
Aspect 22. The system of aspect 21, wherein the control unit is further configured to operate the sample manipulation device so as to apply a force to the sample matrix sufficient to move specifically-bound analyte on the sensing element and thereby manipulate at least a portion of specifically-bound analyte on the sensing element; and wherein the property determination unit is configured to determine the property of the analyte in the sample further based on the measurement signal during and/or after the control unit operates the sample manipulation device to manipulate specifically-bound analyte.
Aspect 23. The system of either of aspect 21 or aspect 22, wherein the control unit is further configured to operate the sample manipulation device so as to apply a force to the sample matrix sufficient to detach specifically-bound analyte on the sensing element; and wherein the property determination unit is configured to determine the property of the analyte in the sample further based on the measurement signal during and/or after the control unit operates the sample manipulation device to detach specifically-bound analyte.
Aspect 24. The method of aspects 23, wherein the control unit is configured to operate the sample manipulation device so as to apply a force to the sample matrix sufficient to detach specifically-bound analyte by applying a first force and increasing the applied force to a second force, optionally wherein the first force is from 0 to 50 pN and the second force is greater than the first force by at least 20 pN.
Aspect 25. The system of any of aspects 22 to 24, wherein the sample manipulation device generates an electric field and/or a magnetic field to provide the force.
Aspect 26. The system of any of aspects 21 to 25, wherein the sensing element comprises a sensing layer, the sensing layer having a plurality of structures formed therein; and wherein the measurement signal is dependent on the location of analyte relative to the plurality of structures in the sensing layer.
Aspect 27. The system of aspect 26, wherein the measurement signal is indicative of an impedimetric property of the sensing layer; and wherein the plurality of structures are arranged on or in the sensing layer such that an analyte received between or within the structures modifies the impedimetric property.
Aspect 28. The system of either of aspect 26 or aspect 27, wherein the plurality of structures comprise a plurality of wells formed in the sensing layer and/or a plurality of through holes extending from a first surface of the sensing layer to a second surface of the sensing layer.
Aspect 29. The system of any of aspects 21 to 28, wherein the plurality of electrodes configured to provide the electric field comprises at least a first electrode provided above the sensing layer and a second electrode provided below the sensing layer.
Aspect 30. The system of any of aspects 21 to 29, wherein the control unit is configured to operate the sample manipulation device to generate the electric field so as remove any non-specifically-bound analyte and/or non-analyte species but retain specifically-bound analyte on the sensing element by applying a first force and subsequently increasing the applied force to a second force, optionally wherein the first force is from 0 to 20 pN and the second force is greater than the first force by at least 20 pN.

## Claims

1. A method for determining a property of an analyte in a sample, the sample comprising the analyte, non-analyte species and a sample matrix in which the analyte and non-analyte species are contained, the method comprising:
providing a sensor assembly, the sensor assembly comprising a sensing element comprising a capture species configured to specifically bind with the analyte, the sensing element providing a measurement signal indicative of the interaction of the sensing element with the sample;
providing the sample to the sensing element, wherein at least a portion of analyte and/or non-analyte species in the sample non-specifically bind to the sensing element and wherein least a portion of analyte specifically-binds to the sensing element;
obtaining a sample baseline measurement based on the measurement signal;
removing at least a portion of the non-specifically-bound analyte and/or non-analyte species from the sensing element by applying an electric field to the sample matrix, the electric field configured to remove non-specifically-bound analyte and/or non-analyte species from at least a portion of the sensing element but having a strength less than that required to detach specifically-bound analyte from the sensing element; and
determining the property of the analyte in the sample based on the measurement signal after the step of removing at least a portion of the non-specifically-bound analyte and/or non-analyte species from the sensing element.

2. The method of claim 1, wherein the sample baseline measurement is used to determine at least one parameter of the step of removing at least a portion of the non-specifically-bound analyte and/or non-analyte species from the sensing element.

3. The method of claim 1 or claim 2, further comprising determining an adjustment factor based on the measurement signal during the step of removing at least a portion of the non-specifically-bound analyte and/or non-analyte species from the sensing element and after the sample baseline measurement, and further comprising adjusting at least one parameter of the step of removing at least a portion of the non-specifically-bound analyte and/or non-analyte species from the sensing element based on the adjustment factor.

4. The method of claim 3, wherein the adjustment factor is based on the rate of change in the measurement signal during the step of removing at least a portion of the non-specifically-bound analyte and/or non-analyte components from the sensing element.

5. The method of any preceding claim, further comprising:
subsequently providing a second sample to the sensor assembly, the sample comprising analyte, non-analyte species and a sample matrix in which the analyte and non-analyte species are contained; and
determining the property of the analyte in the second sample based on the measurement signal for the second sample and a signal compensation factor, wherein the signal compensation factor is based on the sample baseline measurement and the measurement signal for the sample.

6. The method of any preceding claim, wherein the sample baseline measurement is obtained at least during the step of removing at least a portion of the non-specifically-bound analyte and/or non-analyte species from the sensing element, and the sample baseline measurement comprises the rate of change in the measurement signal.

7. The method of any preceding claim, wherein the strength of the electric field is varied during the step of removing at least a portion of the non-specifically-bound analyte and/or non-analyte components from the sensing element.

8. The method of any preceding claim,
further comprising manipulating at least a portion of the specifically-bound analyte after the step of removing at least a portion of the non-specifically-bound analyte and/or non-analyte components from the sensing element, wherein manipulating specifically-bound analyte comprises applying a force to the sample matrix sufficient to move specifically-bound analyte on the sensing element; and
wherein determining the property of the analyte in the sample is based on the measurement signal during and/or after the step of manipulating specifically-bound analyte,
optionally wherein determining the property of the analyte is based on the rate of change in the measurement signal during the step of manipulating specifically-bound analyte.

9. The method of any preceding claim,
further comprising detaching at least a portion of the specifically-bound analyte after the step of removing at least a portion of the non-specifically-bound analyte and/or non-analyte components from the sensing element, wherein detaching specifically-bound analyte comprises applying a force to the sample matrix sufficient to detach specifically-bound analyte from the sensing element; and
wherein determining the property of the analyte in the sample is based on the measurement signal during and/or after the step of detaching the specifically-bound analyte,
optionally wherein determining the property of the analyte is based on the rate of change in the measurement signal during the step of detaching the specifically-bound analyte.

10. The method of any preceding claim,
wherein the method further comprises providing a detection species to the sample matrix, the detection species specifically-binding to the analyte; and
wherein strength of the electric field configured to remove non-specifically-bound analyte and/or non-analyte species from at least a portion of the sensing element is less than that required to detach specifically-bound detection species from the analyte.

11. The method of any preceding claim, wherein the sensing element comprises a sensing layer, the sensing layer having a plurality of structures formed therein; and wherein the measurement signal is dependent on the location of the analyte relative to the plurality of structures in the sensing layer,
optionally wherein the measurement signal is indicative of an impedimetric property of the sensing layer and the configuration of the sensing layer and the capture species is such that analyte that is specifically bound to the capture species is able to modify an impedimetric property of the sensing layer.

12. A system for determining a property of an analyte in a sample, the sample comprising the analyte, non-analyte species and a sample matrix in which the analyte and non-analyte species are contained, the system comprising:
a sensor assembly comprising a sensing element, the sensing element comprising a capture species configured to specifically bind with the analyte and the sensing element providing a measurement signal indicative of the interaction of the sensing element with the sample;
a sample manipulation device for manipulating analyte and/or non-analyte species on the sensing element, the sample manipulation device comprising a plurality of electrodes configured to apply an electric field about the sensing element;
a control unit configured to operate the sample manipulation device; and
a property determination unit configured to determine the property of the analyte,
wherein the control unit is configured to operate the sample manipulation device to generate the electric field so as remove any non-specifically-bound analyte and/or non-analyte species but retain specifically-bound analyte on the sensing element;
wherein the property determination unit is configured to determine a sample baseline measurement based on the measurement signal before and/or during operation of the sample manipulation device; and
wherein the property determination unit is configured to determine the property of the analyte based on the measurement signal after operation of the sample manipulation device.

13. The system of claim 12, wherein the control unit is further configured to operate the sample manipulation device so as to apply a force to the sample matrix sufficient to move specifically-bound analyte on the sensing element and thereby manipulate at least a portion of specifically-bound analyte on the sensing element; and
wherein the property determination unit is configured to determine the property of the analyte in the sample further based on the measurement signal during and/or after the control unit operates the sample manipulation device to manipulate specifically-bound analyte.

14. The system of claim 12 or claim 13, wherein the control unit is further configured to operate the sample manipulation device so as to apply a force to the sample matrix sufficient to detach specifically-bound analyte on the sensing element; and
wherein the property determination unit is configured to determine the property of the analyte in the sample further based on the measurement signal during and/or after the control unit operates the sample manipulation device to detach specifically-bound analyte.

15. The system of claim 14, wherein the sensing element comprises a sensing layer, the sensing layer having a plurality of structures formed therein; and
wherein the measurement signal is dependent on the location of analyte relative to the plurality of structures in the sensing layer.
